(19) Europäisches Patentamt
European Patent Office
Office européen des brevets

(11) Veröffentlichungsnummer: **0 332 025 B1**

(12) # EUROPÄISCHE PATENTSCHRIFT

(45) Veröffentlichungstag der Patentschrift: **23.06.93**

(51) Int. Cl.5: **C07C 255/18**, C07C 255/11, C07C 255/16, C07C 43/225, C07C 69/712, C07C 69/63, C07C 69/92, C07D 239/26, C07D 213/30, C09K 19/12, C09K 19/20

(21) Anmeldenummer: **89103497.7**

(22) Anmeldetag: **28.02.89**

Verbunden mit 89903152.0/0357739 (europäische Anmeldenummer/Veröffentlichungsnummer) durch Entscheidung vom 22.11.91.

Die Akte enthält technische Angaben, die nach dem Eingang der Anmeldung eingereicht wurden und die nicht in dieser Patentschrift enthalten sind.

(54) **Chirale Derivate des 1,2 -Difluorbenzols.**

(30) Priorität: **10.03.88 DE 3807802**

(43) Veröffentlichungstag der Anmeldung:
**13.09.89 Patentblatt 89/37**

(45) Bekanntmachung des Hinweises auf die Patenterteilung:
**23.06.93 Patentblatt 93/25**

(84) Benannte Vertragsstaaten:
**CH DE ES FR GB GR IT LI LU NL SE**

(56) Entgegenhaltungen:
**EP-A- 0 188 222**
**EP-A- 0 256 303**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Reiffenrath, Volker**
**Jahnstrasse 18**
**W-6101 Rossdorf(DE)**
Erfinder: **Krause, Joachim, Dr.**
**Samuel-Morse-Strasse 14**
**W-6110 Dieburg(DE)**
Erfinder: **Wächtler, Andreas Dr.**
**Goethestrasse 34**
**W-6103 Griesheim(DE)**
Erfinder: **Geelhaar, Thomas, Dr.**
**Trajanstrasse 12**
**W-6500 Mainz(DE)**
Erfinder: **Bartmann, Ekkehard, Dr.**
**Dieburger Strasse 12a**
**W-6106 Erzhausen(DE)**
Erfinder: **Hittich, Reinhard, Dr.**
**Am Kirchberg 11**
**W-6101 Modautal(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 332 025 B1

## Beschreibung

Die Erfindung betrifft chirale Derivate des 1,2-Difluorbenzols der Formel I,

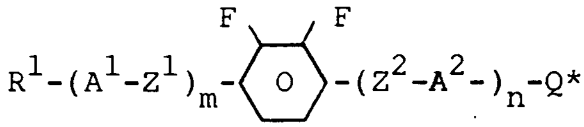

I

worin

- $R^1$ H, F, Cl, Br, CN, eine Alkyl- oder Perfluoralkyl-Gruppe mit jeweils 1 bis 12 C-Atomen, worin auch eine oder zwei nicht benachbarte $CH_2$- bzw. $CF_2$-Gruppen durch O-Atome und/oder -CO-Gruppen und/oder -CO-O-Gruppen und/oder -CH=CH-Gruppen und/oder -CHHalogen und/oder -CO-O-CHCN-Gruppen ersetzt sein können, oder einen optisch aktiven Rest der Formel

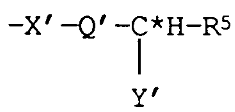

worin

- X' -CO-O-, -O-CO-, -O-CO-O, -CO-, -O-, -S-, -CH=CH-, -CH=CH-COO- oder eine Einfachbindung,
- Q' Alkylen mit 1 bis 5 C-Atomen, worin auch eine nicht mit X' verknüpfte $CH_2$-Gruppe durch -O-, -CO-, -O-CO- oder -CH=CH-ersetzt sein kann, oder eine Einfachbindung,
- Y' CN, Halogen oder Methyl, und
- $R^5$ eine von Y' verschiedene Alkylgruppe mit 1 bis 15 C-Atomen, worin auch eine oder zwei nicht benachbarte $CH_2$-Gruppen durch -O-, -CO-, -O-CO-, -CO-O- und/oder -CH=CH- ersetzt sein können,

- $A^1$ und $A^2$ jeweils unabhängig voneinander unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N ersetzt sein können, 1,4-Cyclohexylen, worin auch eine oder zwei nicht benachbarte $CH_2$-Gruppen durch O-Atome und/oder S-Atome ersetzt sein können, Piperidin-1,4-diyl, 1,4-Bicyclo(2,2,2)octylen, 1,3,4-Thiadiazol-2,5-diyl, Naphthalin-2,6-diyl, Decahydronaphthalin-2,6-diyl- oder 1,2,3,4-Tetrahydronaphthalin-2,6-diyl,
- $Z^1$ und $Z^2$ jeweils -CO-O, -O-CO-, $-CH_2CH_2-$, $-OCH_2-$, $-CH_2O-$, -C≡C- oder eine Einfachbindung,
- m 0, 1 oder 2,
- n 0, 1 oder 2,
- (m + n) 1 oder 2 und
- Q* einen Chiralität induzierenden organischen Rest mit einem asymmetrischen Kohlenstoffatom bedeutet.

Die Verbindungen der Formel I können wie ähnliche in DE-OS 35 15 373 beschriebene Verbindungen als Komponenten chiraler getilteter smektischer flüssigkristalliner Phasen verwendet werden.

In den europäischen Patentanmeldungen EP 0 188 222 und EP 0 256 303 werden optisch aktive Phenyl-, Biphenyl-benzoate und Phenyl-biphenylcarboxylate mit nur einem lateralen Fluoratom zur Verwendung als ferroelektrische Flüssigkristalle vorgeschlagen. Keinem dieser Dokumente kann ein Hinweis entnommen werden, daß solche Substanzen zum Dotieren achiraler Basismaterialien eingesetzt werden können.

Der technischen Lehre dieser Dokumente kann der Fachmann nur entnehmen, daß man die dort beschriebenen Verbindungen zur Herstellung ferroelektrischer Mischungen auch mit anderen optisch aktiven Substanzen mischen kann.

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982);

H.R. Brand et al., J. Physique 44, (lett.), L-771 (1983). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiralen getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 µm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hinund hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiralen getilteten smektischen Phasen (wie z.B. Sc*) ist deren relativ hohe optische Anisotropie, die durch relativ hohe Viskositätswerte bedingten nicht ausreichend kurzen Schaltzeiten, sowie, daß die dielektrische Anisotropie Werte größer Null oder, falls negativ, nur wenig von Null verschiedene Werte aufweist. Negative Werte der dielektrischen Anisotropie sind erforderlich, falls die erforderliche planare Orientierung durch Überlagerung des Ansteuerfeldes mit einem AC-Haltefeld mit kleiner Amplitude bewirkt wird (J.M. Geary, SID-Tagung, Orlando/Florida, April/Mai 1985, Vortrag 8.3).

Es wurde nun gefunden, daß die Verwendung von Verbindungen der Formel I als Komponenten chiraler getilteter smektischer Mischungen die erwähnten Nachteile wesentlich vermindern kann. Die Verbindungen der Formel I sind somit als Komponenten chiraler getilteter smektischer flüssigkristalliner Phasen vorzüglich geeignet. Insbesondere sind mit ihrer Hilfe chemisch besonders stabile chirale getiltete smektische flüssigkristalline Phasen mit günstigen ferroelektrischen Phasenbereichen, insbesondere mit breiten Sc*-Phasenbereichen, negativer oder auch positiver dielektrischer Anisotropie, niedriger optischer Anisotropie, günstiger Pitchhöhe, niedriger Viskosität und für derartige Phasen hohen Werten für die spontane Polarisation und sehr kurzen Schaltzeiten herstellbar. P ist die spontane Polarisation in $nC/cm^2$.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung ferroelektrischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die spontane Polarisation und/oder den Phasenbereich und/oder den Tiltwinkel und/oder den Pitch und/oder die Schaltzeiten einer solchen Phase zu variieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und weisen günstige Werte der optischen Anisotropie auf. Teilweise zeigen die Verbindungen der Formel I flüssigkristalline Mesophasen in einem für die elektrooptische Verwendung günstig gelegenen Temperaturbereich, es können jedoch auch isotrope oder monotrop flüssigkristalline Verbindungen der Formel I als Komponenten chiraler getilteter smektischer Phasen vorteilhaft eingesetzt werden. Chemisch, thermisch und gegen Licht sind sie sehr stabil.

Gegenstand der Erfindung sind somit die Verbindungen der Formel I sowie die Verwendung der Verbindungen der Formel I als Komponenten flüssigkristalliner Phasen.

Gegenstand der Erfindung sind auch chirale getiltete smektische flüssigkristalline Phasen mit einem Gehalt an mindestens einer Verbindung der Formel I mit mindestens einem mit vier verschiedenen Substituenten verknüpften Kohlenstoffatom.

Gegenstand der Erfindung sind ferner solche Phasen mit einem Gehalt an mindestens einer Verbindung der Formel I sowie Flüssigkristallanzeigeelemente, insbesondere ferroelektrische elektrooptische Anzeigeelemente, die derartige Phasen enthalten.

Der Einfachheit halber bedeuten im folgenden Ph eine unsubstituierte oder durch ein oder zwei Fluor substituierte 1,4-Phenylengruppe, worin auch eine oder zwei CH-Gruppen durch N ersetzt sein können, Cy eine 1,4-Cyclohexylengruppe, worin auch eine oder zwei nicht benachbarte $CH_2$-Gruppen durch O-Atome

ersetzt sein können, Thi eine 1,3,4-Thiadiazol-2,5-diylgruppe und Bi eine Bicyclo(2,2,2)octylengruppe. $PheF_2$ ist eine Gruppe der Formel

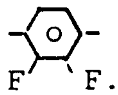

Vor- und nachstehend haben $Q^*$, $A^1$, $A^2$, $Z^1$, $Z^2$, m und n die angegebene Bedeutung, sofern nicht ausdrücklich etwas anderes vermerkt ist.

Die Verbindungen der Formel I umfassen dementsprechend insbesondere Verbindungen der Teilformeln Ia bis Id (mit zwei Ringen)

$R^1$-$PheF_2$-$A^2$-$Q^*$ Ia

$R^1$-$PheF_2$-$Z^2$-$A^2$-$Q^*$ Ib

$Q^*$-$PheF_2$-$A^1$-$R^1$ Ic

$Q^*$-$PheF_2$-$Z^1$-$A^1$-$R^1$ Id

und Ie bis It (mit drei Ringen):

$R^1$-$PheF_2$-$A^2$-$A^2$-$Q^*$ Ie

$R^1$-$PheF_2$-$Z^2$-$A^2$-$A^2$-$Q^*$ If

$R^1$-$PheF_2$-$A^2$-$Z^2$-$A^2$-$Q^*$ Ig

$R^1$-$PheF_2$-$Z^2$-$A^2$-$Z^2$-$A^2$-$Q^*$ Ih

$R^1$-$A^1$-$PheF_2$-$A^2$-$Q^*$ Ii

$R^1$-$A^1$-$Z^1$-$PheF_2$-$A^2$-$Q^*$ Ij

$R^1$-$A^1$-$PheF_2$-$Z^2$-$A^2$-$Q^*$ Ik

$R^1$-$A^1$-$Z^1$-$PheF_2$-$Z^2$-$A^2$-$Q^*$ Il

$Q^*$-$PheF_2$-$A^1$-$A^1$-$R^1$ Im

$Q^*$-$PheF_2$-$Z^1$-$A^1$-$A^1$-$R^1$ In

$Q^*$-$PheF_2$-$A^1$-$Z^1$-$A^1$-$R^1$ Io

$Q^*$-$PheF_2$-$Z^1$-$A^1$-$Z^1$-$A^1$-$R^1$ Ip

$Q^*$-$A^2$-$PheF_2$-$A^1$-$R^1$ Iq

$Q^*$-$A^2$-$Z^2$-$PheF_2$-$A^1$-$R^1$ Ir

$Q^*$-$A^2$-$PheF_2$-$Z^1$-$A^1$-$R^1$ Is

$Q^*$-$A^2$-$Z^2$-$PheF_2$-$Z^1$-$A^1$-$R^1$ It

Darunter sind diejenigen der Formeln Ia, Ic, Ie, Im, In und If besonders bevorzugt.

Besonders bevorzugt sind Verbindungen der Formel I'

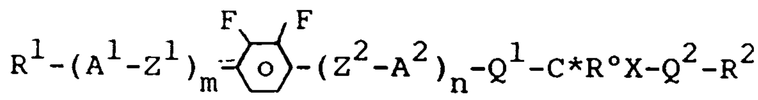 I'

worin $Q^1$, $Q^2$, R° und X die in Anspruch 2 angegebene Bedeutung haben. R° ist eine von X und $Q^2$-$R^2$ verschiedene Alkylgruppe mit vorzugsweise 1 bis 5 C-Atomen. Besonders bevorzugt sind Methyl und Ethyl, insbesondere Methyl. $R^2$ ist eine Alkylgruppe mit 2 bis 10, insbesondere mit 2 bis 6, 6-Atomen. $Q^1$ und $Q^2$ bedeuten vorzugsweise jeweils unabhängig voneinander -O-CO- (wobei das Carbonylkohlenstoffatom mit dem asymmetrischen C-Atom C* verknüpft ist), -O-$CH_2$-(wobei die Methylengruppe mit dem asymmetrischen C-Atom C* verknüpft ist), -$CH_2CH_2$-, -$CH_2$- oder eine Einfachbindung (-). Besonders bevorzugte Kombinationen von $Q^1$ und $Q^2$ sind in der folgenden Tabelle angegeben:

| $Q^1$ | -O-CO- | -O-$CH_2$- | -$CH_2$- | -$CH_2CH_2$- | -$CH_2$- | -$CH_2CH_2$- |
|---|---|---|---|---|---|---|
| $Q^2$ | - | - | -CO-O- | -CO-O- | -$CH_2$-O- | -$CH_2$-O- |

In den bevorzugten Verbindungen der vor- und nachstehenden Formeln können die Alkylreste, in denen auch eine $CH_2$-Gruppe (Alkoxy bzw. Oxaalkyl) durch ein 0-Atom ersetzt sein kann, geradkettig oder verzweigt sein. Vorzugsweise haben sie 5, 6, 7, 8, 9 oder 10 C-Atome und bedeuten demnach bevorzugt Pentyl, Hexyl, Heptyl, Octyl, Nonyl, Decyl, Pentoxy, Hexoxy, Heptoxy, Octoxy, Nonoxy oder Decoxy, ferner auch Ethyl, Propyl, Butyl, Undecyl, Dodecyl, Propoxy, Ethoxy, Butoxy, Undecoxy, Dodecoxy, 2-Oxapropyl (= 2-Methoxymethyl), 2- (= Ethoxymethyl) oder 3-Oxabutyl (= 2-Methoxypentyl), 2-, 3- oder 4-Oxapentyl, 2-, 3-, 4- oder 5-Oxahexyl, 2-, 3-, 4-, 5- oder 6-Oxaheptyl.

$A^1$ und $A^2$ sind bevorzugt Cy oder Ph. In den Verbindungen der vor- und nachstehenden Formeln bedeutet Ph vorzugsweise eine 1,4-Phenylen- (Phe), eine Pyrimidin-2,5-diyl- (Pyr), eine Pyridin-2,5-diyl- (Pyn), eine Pyrazin-3,6-diyl- oder eine Pyridazin-2,5-diyl-Gruppe, insbesondere bevorzugt Phe, Pyr oder Pyn. Vorzugsweise enthalten die erfindungsgemäßen Verbindungen nicht mehr als eine 1,4-Phenylengruppe, worin eine oder zwei CH-Gruppen durch N ersetzt sind. Cy bedeutet vorzugsweise eine 1,4-Cyclohexylengruppe. Insbesondere bevorzugt sind jedoch Verbindungen der Formel I, worin $A^2$, eine in 1- oder 4-Position durch CN substituierte 1,4-Cyclohexylengruppe bedeutet und die Nitrilgruppe sich in axialer Position befindet, d.h. die Gruppe $A^2$ die folgende Konfiguration aufweist:

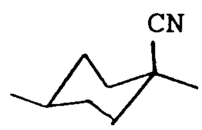

Besonders bevorzugt sind Verbindungen der Formel I und der vorstehenden Teilformeln, die eine Gruppierung -Ph-Ph- enthalten. -Ph-Ph- ist vorzugsweise -Phe-Phe-, Phe-Pyr oder Phe-Pyn. Besonders bevorzugt sind die Gruppen

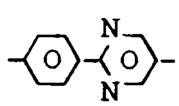 und 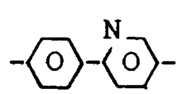 ,

sowie
ferner unsubstituiertes oder ein- oder mehrfach durch Fluor substituiertes 4,4'-Biphenylyl.

$Z^1$ und $Z^2$ sind bevorzugt Einfachbindungen, in zweiter Linie bevorzugt -0-C0-, -C0-0-, -C≡C- oder -$CH_2CH_2$-Gruppen.

Besonders bevorzugt für $Z^1$ ist -CO-O, -O-CO-, -C≡C- oder -$CH_2CH_2$-, insbesondere die -$CH_2CH_2$- und die -C≡C-Gruppe.

X bedeutet in den Verbindungen der vor- und nachstehenden Formeln Halogen, CN oder $CH_3$, vorzugsweise F, Cl, $CH_3$ oder CN. Besonders bevorzugt sind F und CN.

$R^o$ ist eine von X verschiedene, vorzugsweise geradkettige Alkylgruppe mit vorzugsweise bis zu 5 C-Atomen. Besonders bevorzugt wird Methyl und Ethyl, insbesondere Methyl.

Die bevorzugte Bedeutung von $Q^1$ und $Q^2$ ist Alkylen mit 1 bis 2 C-Atomen, -O-, -O-CO-, -CO-O- und eine Einfachbindung. Ferner bevorzugte Bedeutungen von $Q^1$ bzw. $Q^2$ sind $-CH_2O-$ und $-O-CH_2-$.

Verbindungen der vor- und nachstehenden Formeln mit verzweigten Flügelgruppen $R^1$ können von Bedeutung sein. Verzweigte Gruppen dieser Art enthalten in der Regel nicht mehr als zwei Kettenverzweigungen. $R^1$ ist vorzugsweise eine geradkettige Gruppe oder eine verzweigte Gruppe mit nicht mehr als einer Kettenverzweigung.

Bevorzugte verzweigte Reste sind Isopropyl, 2-Butyl (= 1-Methylpropyl), Isobutyl (= 2-Methylpropyl), tert.-Butyl, 2-Methylbutyl, Isopentyl (= 3-Methylbutyl), 2-Methylpentyl, 3-Methylpentyl, 4-Methylpentyl, 2-Ethylhexyl, 5-Methylhexyl, 2-Propylpentyl, 6-Methylheptyl, 7-Methyloctyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Der Rest $R^1$ kann auch ein optisch aktiver organischer Rest mit einem asymmetrischen Kohlenstoffatom sein. Vorzugsweise ist dann das asymmetrische Kohlenstoffatom mit zwei unterschiedlich substituierten C-Atomen, einem H-Atom und einem Substituenten ausgewählt aus der Gruppe Halogen (insbesondere F, Cl oder Br), Alkyl oder Alkoxy mit jeweils 1 bis 5 C-Atomen und CN verknüpft. Der optisch aktive organische Rest $R^1$ hat vorzugsweise die Formel,

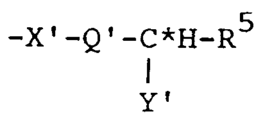

worin

- X' -CO-O-, -O-CO-, -O-CO-O-, -CO-, -O-, -S-, -CH=CH-, -CH=CH-COO- oder eine Einfachbindung,
- Q' Alkylen mit 1 bis 5 C-Atomen, worin auch eine nicht mit X' verknüpfte $CH_2$-Gruppe durch -O-, -CO-, -O-CO-, -CO-O- oder -CH=CH- ersetzt sein kann, oder eine Einfachbindung,
- Y' CN, Halogen oder Methyl und
- $R^5$ eine von Y verschiedene Alkylgruppe mit 1 bis 15 C-Atomen, worin auch eine oder zwei nicht benachbarte $CH_2$-Gruppen durch -O-, -CO-, -O-CO-, -CO-O- und/oder -CH=CH- ersetzt sein können,

bedeutet.

- X' ist vorzugsweise -CO-O-, -O-CO-, -O-, -CH=CH-COO-(trans) oder eine Einfachbindung. Besonders bevorzugt sind -O- oder eine Einfachbindung.
- Q' ist vorzugsweise $-CH_2-$, $-CH_2CH_2-$, $-CH_2CH_2CH_2-$ oder eine Einfachbindung, insbesondere bevorzugt eine Einfachbindung, $-CH_2-$ und $-CH_2CH_2-$.
- Y' ist vorzugsweise $CH_3$, -CN, F oder Cl, insbesondere bevorzugt CN oder F.
- $R^5$ ist vorzugsweise geradkettiges oder verzweigtes Alkyl mit 1 bis 10, insbesondere mit 1 bis 7, C-Atomen.

Besonders bevorzugt sind die folgenden 5 Kombinationen von X', Q', Y' und $R^5$:

| | | | | | |
|---|---|---|---|---|---|
| X' | -O- | - | -O- oder -CO-O- | -COO-oder -O- | -OOC- |
| Q' | $-CH_2-$ | $-CH_2CH_2-$ | - | - | - |
| Y' | F | F | $CH_3$ | CN | F oder Cl |
| $R^5$ | -Alkyl | -Alkyl | -COO-Alkyl | Alkyl | Alkyl |

Unter den Verbindungen der Formel I sowie Ia bis Ii sind diejenigen bevorzugt, in denen mindestens einer der darin enthaltenen Reste eine der angegebenen bevorzugten Bedeutungen hat.

In den Verbindungen der Formel I sowie in den vor- und nachstehenden Teilformeln ist $-(A^1-Z^1)_m-PheF_2-(Z^2-A^2)_n-$vorzugsweise eine Gruppe der folgenden Formeln 1 bis 16 oder deren Spiegelbild:

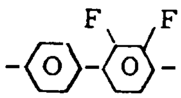
1

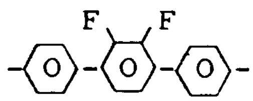
2

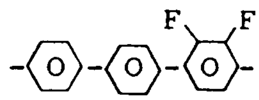
3

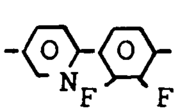
4

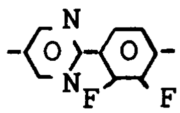
5

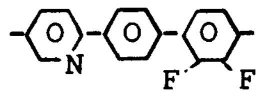
6

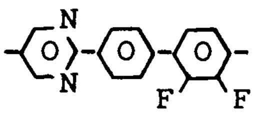
7

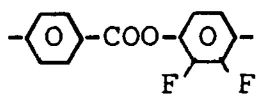
8

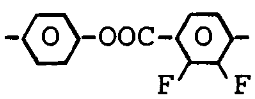
9

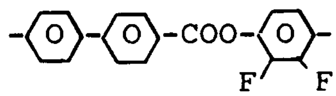
10

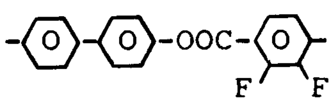
11

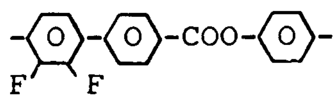
12

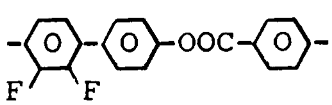
13

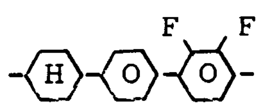
14

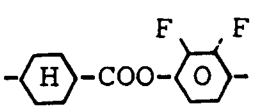
15

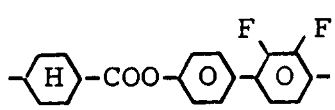
16

Gruppen der Formeln 1, 3, 4, 5, 7, 8, 10, 11, 12 und 13, insbesondere diejenigen der Formeln 1, 3, 4, 5 und 10-13, sind besonders bevorzugt.

Diejenigen der vorstehend genannten Formeln, die eine oder mehrere Gruppen Dio, Dit, Pip und/oder Pyr enthalten, umschließen jeweils die beiden möglichen 2,5-(Dio, Dit, Pyr) bzw. 1,4-Stellungsisomeren (Pip).

Eine kleinere Gruppe von besonders bevorzugten Dotierstoffen ist diejenige der Formeln

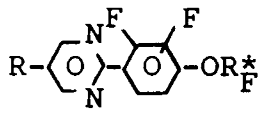

A

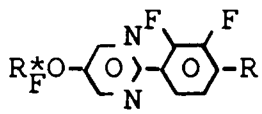

B

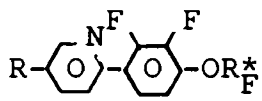

C

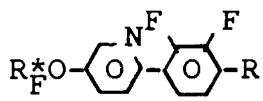

D

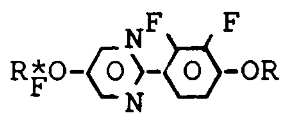

E

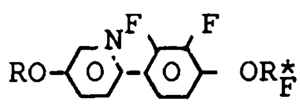

F

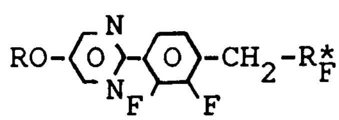

G

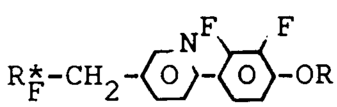

H

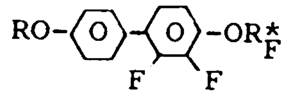

I

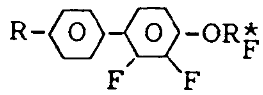

K

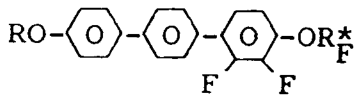

L

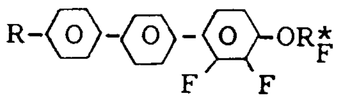

M

$R_F^*$ bedeutet in diesen Formeln geradkettiges oder einfach verzweigtes (vorzugsweise Methylverzweigung) Alkyl mit 3 bis 12 C-Atomen, worin eine $CH_2$-Gruppe durch -$\overset{*}{C}$HF-ersetzt ist und C* ein asymmetrisches C-Atom ist. Vorzugsweise ist

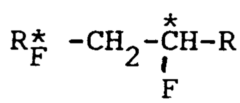

worin R' geradkettiges oder einfach verzweigtes (vorzugsweise Methylverzweigung) Alkyl mit vorzugsweise 2 bis 10, insbesondere 3 bis 10, C-Atomen ist. R hat eine der Bedeutungen für $R^1$ und ist vorzugsweise Alkyl, Oxaalkyl oder Alkenyl mit vorzugsweise 3 bis 12, insbesondere mit 5 bis 12, C-Atomen. Die Gruppen R sind vorzugsweise geradkettig.

Besonders bevorzugt sind ferner Verbindungen der Formel I, worin $-(A^1-Z^1)_m-PheF_2-(Z^2-A^2)_n-$

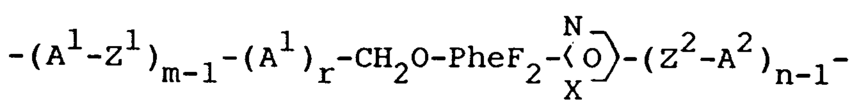

oder

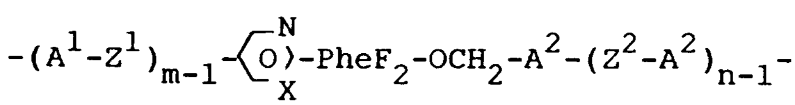

bedeuten.

X ist N oder CH. r ist 0 oder 1.

Besonders bevorzugt sind weiterhin optisch aktive Verbindungen der Formel A'

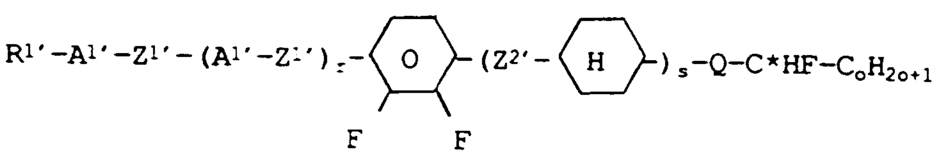

A'

worin

$R^{1'}$ einen unsubstituierten, einen einfach durch -CN oder einen mindestens einfach durch Fluor oder Chlor substituierten Alkyl- oder Alkenylrest mit bis zu 12 C-Atomen, wobei in

| | |
|---|---|
| | diesen Resten auch eine $CH_2$-Gruppe durch -O-, -CO- oder -CO-O- ersetzt sein kann, |
| $A^{1'}$ | jeweils unabhängig voneinander einen unsubstituierten oder durch ein oder zwei Fluoratome substituierten 1,4-Phenylen-Rest, Pyridin-2,5-diyl-Rest, Pyrimidin-2,5-diyl-Rest, Pyrazin-2,5-diyl-Rest, Pyridazin-3,6-diyl-Rest, 1,3,4-Thiadiazol-2,5-diyl-Rest, trans-1,4-Cyclohexylen-Rest, worin auch eine oder zwei nicht benachbarte $CH_2$-Gruppen durch -O- und/oder -S- ersetzt sein können, 1,4-Bicyclo(2.2.2)octylen-Rest oder einen Piperidin-1,4-diyl-Rest, |
| $Z^{1'}$ und $Z^{2'}$ | jeweils unabhängig voneinander -CO-O-, -O-CO-, $-CH_2O-$, $-OCH_2-$, $-CH_2CH_2-$, -C≡C- oder eine Einfachbindung |
| Q | $-OCH_2-$, $-COOCH_2-$ oder $-CH_2OCH_2-$, |
| o | 1 bis 12, |

und einer der beiden Werte

r und s 0 und der andere 0 oder 1 bedeutet.

Die Verbindungen der Formel A können nach an sich bekannten Methoden hergestellt werden, beispielsweise nach oder in Analogie zu folgenden Syntheseschemata:

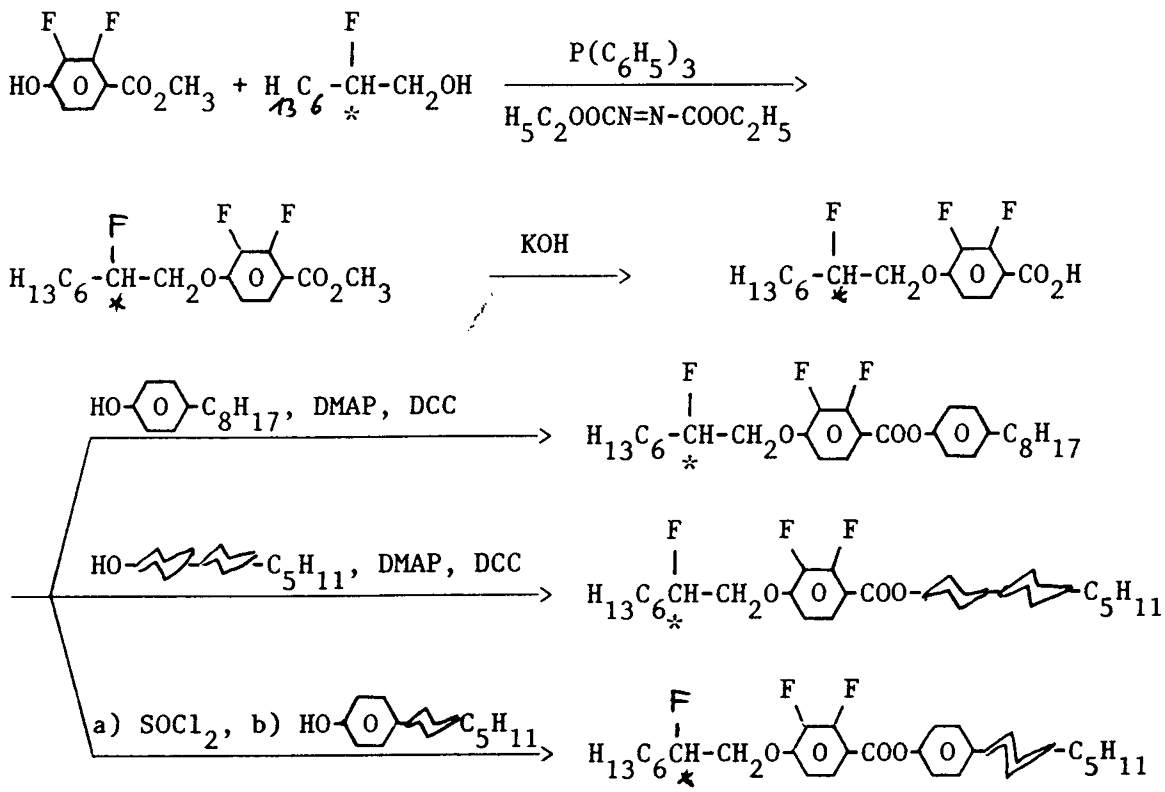


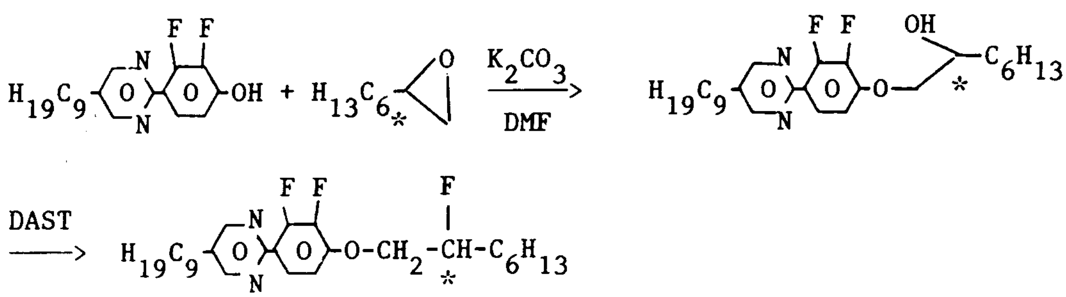

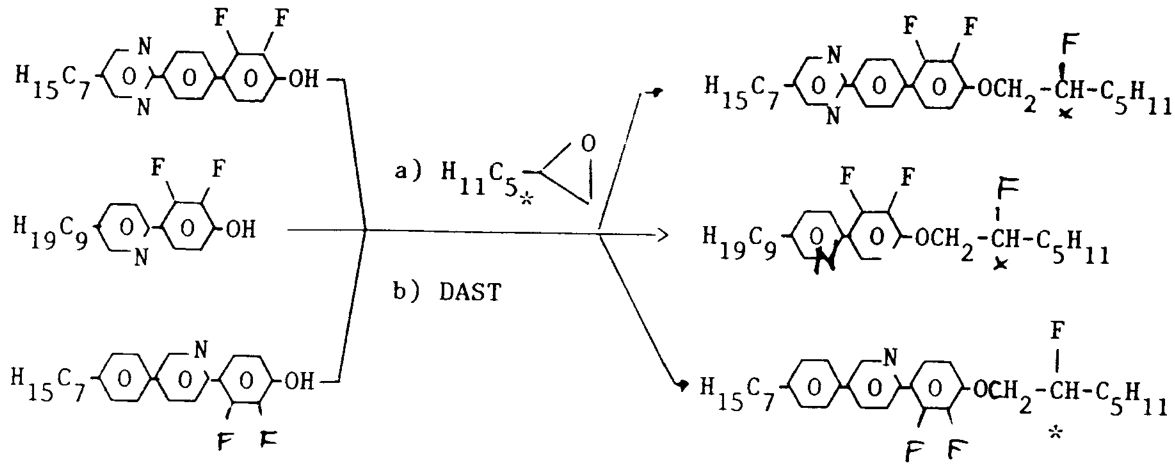


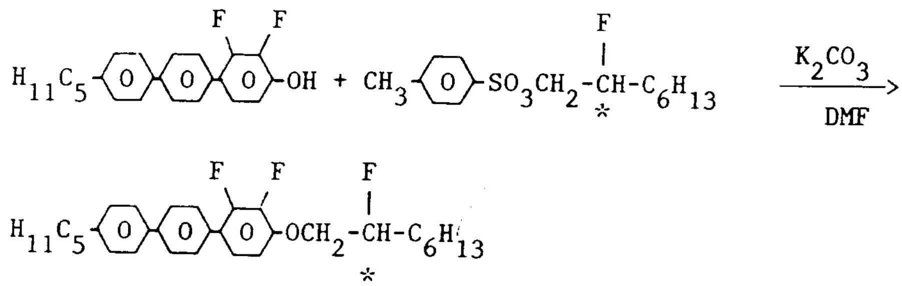


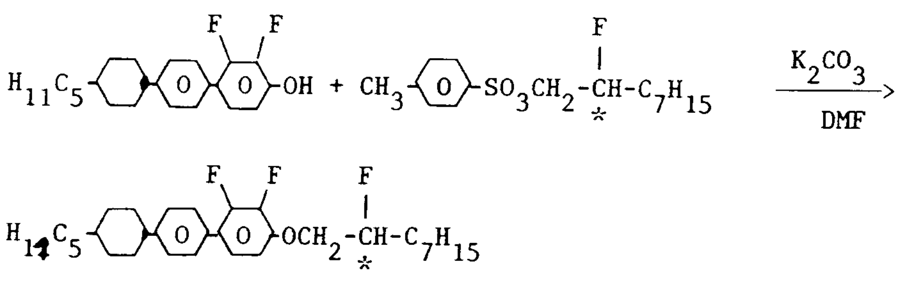


Besonders bevorzugt sind erfindungsgemäße Verbindungen der Teilformel I2'

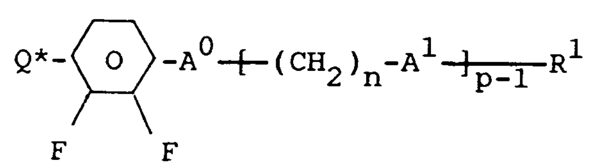

I2'

p ist 1 oder 2. n, $A^1$, Q* und $R^1$ haben die oben angegebene Bedeutung. A ist eine 1,3,4-Thiadiazol-2,5-diyl-, Pyrimidin-2,5-diyl-, Pyridin-2,5-diyl oder Pyrazin-3,6-diyl-Gruppe. Vorzugsweise ist $A^0$

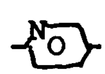 oder 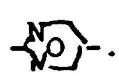.

Eine besonders bevorzugte kleinere Gruppe von Verbindungen ist diejenige der Formel I1'

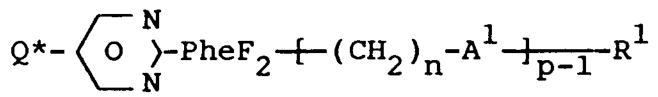

I1'

p ist 1 oder 2. $PheF_2$, n, Q*, $A^1$ und $R^1$ haben die oben angegebene Bedeutung. Die Verbindungen der Formel I1′ können wie im folgenden Schema 1 angegeben hergestellt werden:

Schema 1

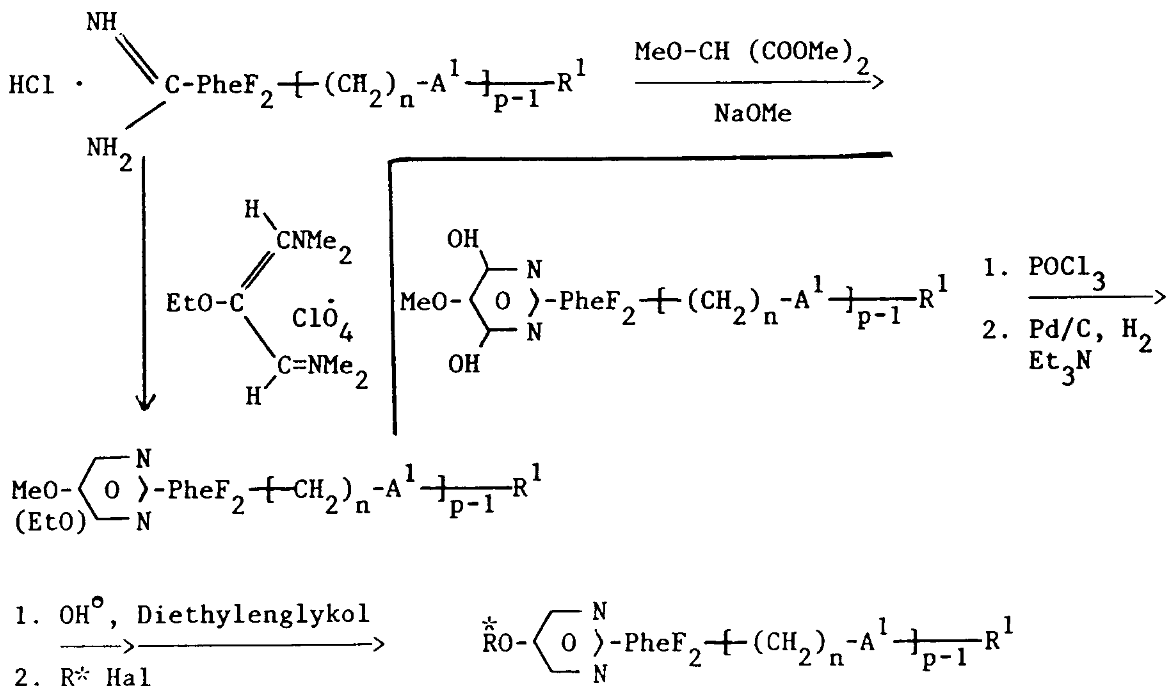

Synthesemöglichkeiten für weitere bevorzugte Verbindungen sind in den folgenden Schemata angegeben:

## Schema 2

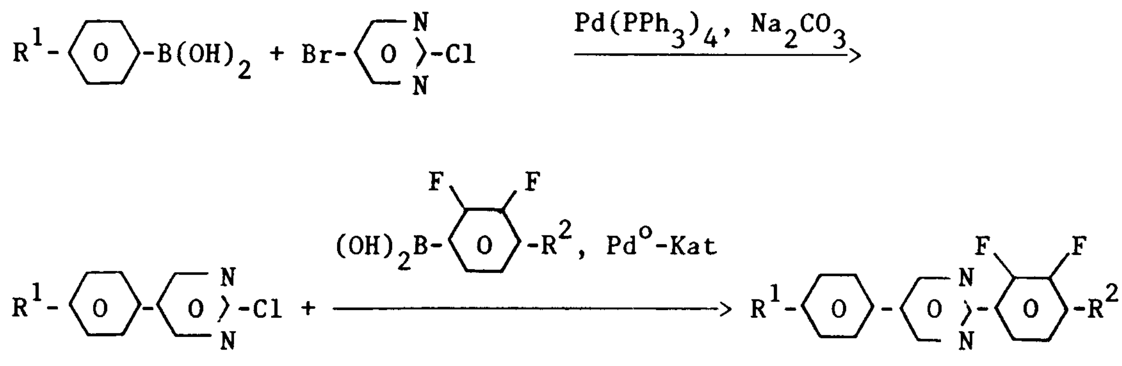


## Schema 3

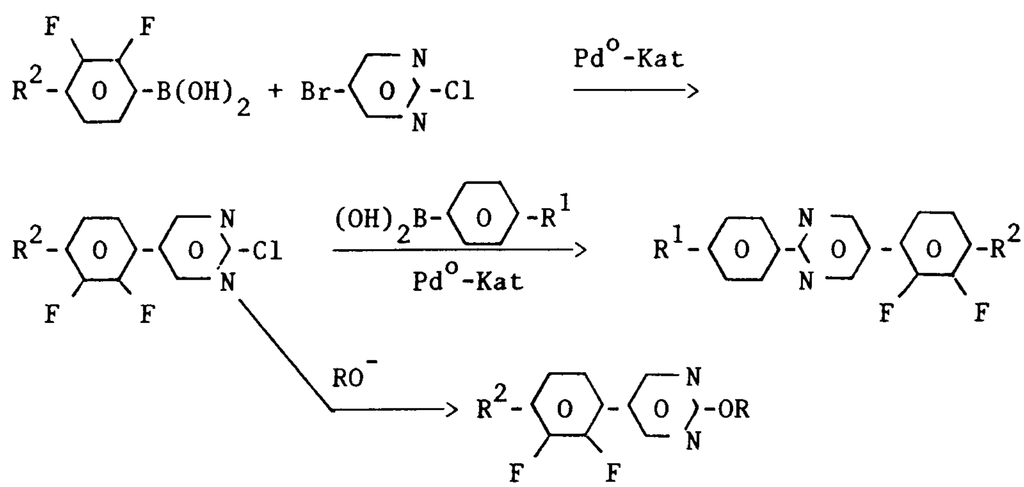

## Schema 4

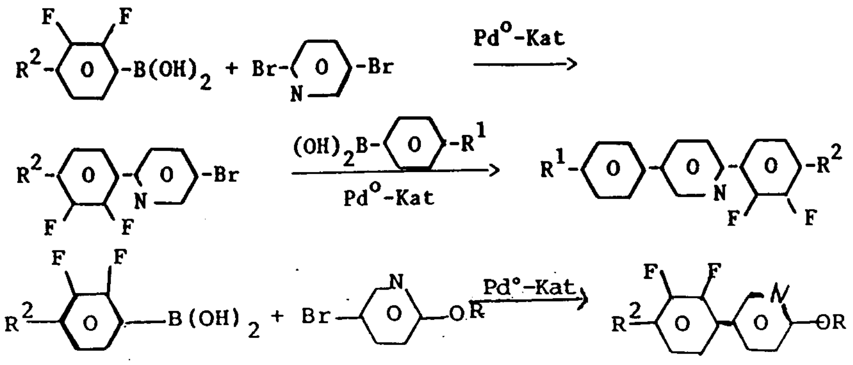


## Schema 5

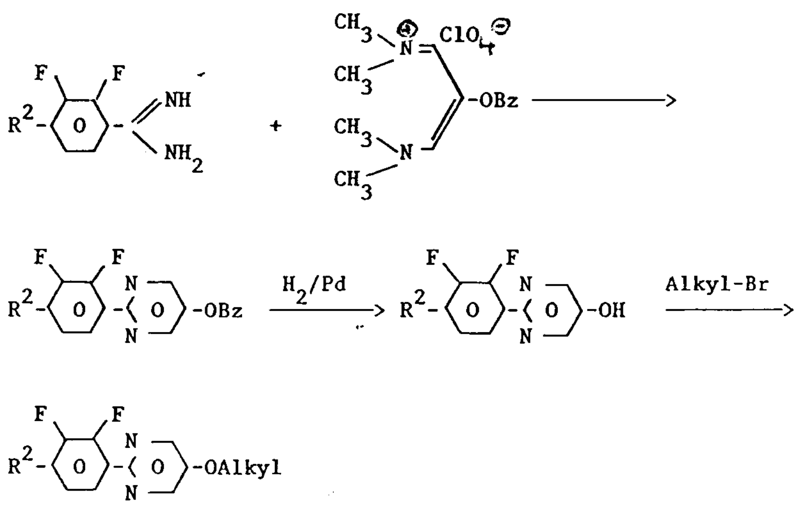


## Schema 6

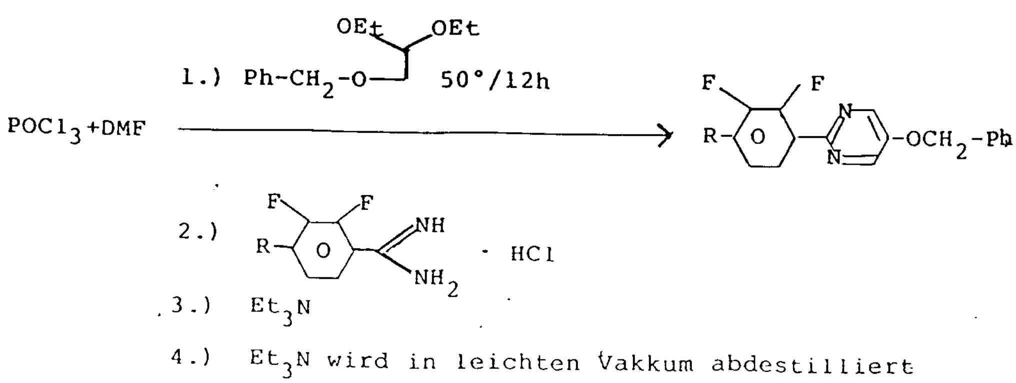

Schema 6

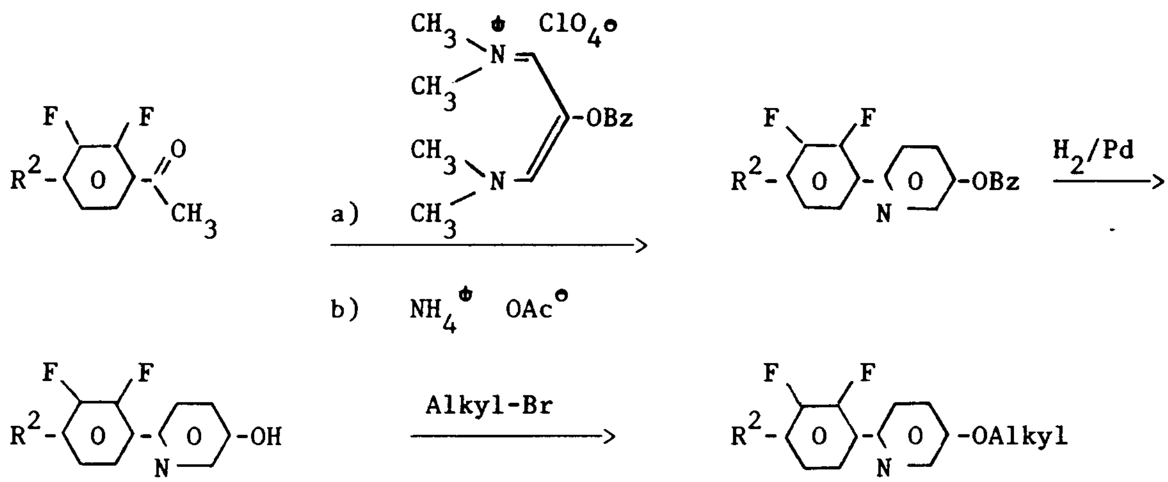


Schema 7

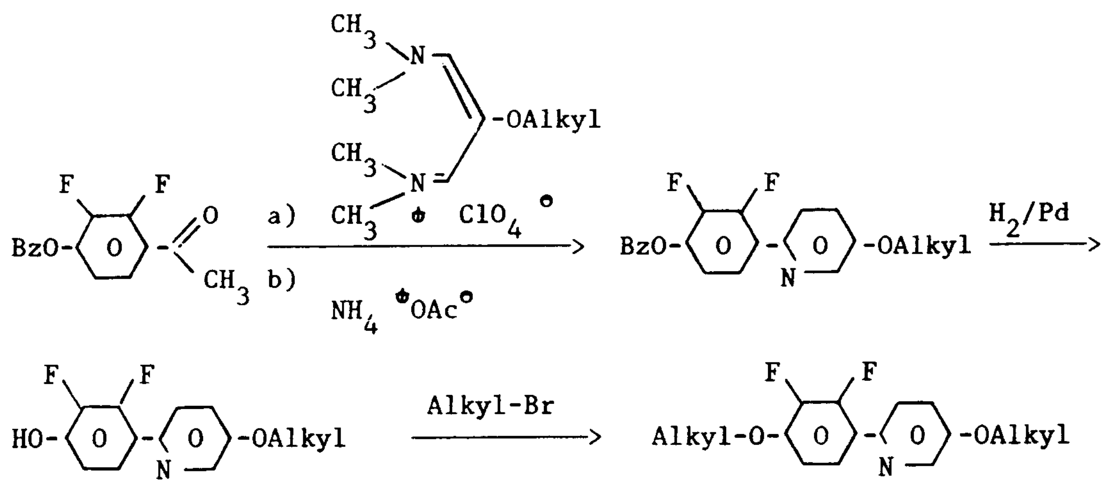


Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Verbindungen der Formel I bzw. deren Vorstufen sind ausgehend von 1,2-Difluorbenzol zugänglich. Dieses wird nach bekanntem Verfahren (z.B. A.M. Roe et al., J. Chem. Soc. Chem. Comm., 22, 582 (1965)) metalliert und mit dem entsprechenden Elektrophil umgesetzt. Mit dem so erhaltenen 1-substituierten 2,3-Difluorbenzol läßt sich diese Reaktionssequenz ein zweites Mal mit einem geeigneten Elektrophil durchführen und man gelangt so zu den für die Synthesen der Verbindungen der Formel I geeigneten 1,4-disubstituierten 2,3-Difluorbenzolen (z.B. Benzoesäuren oder Phenole). 1,2-Difluorbenzol bzw. 1-substituiertes 2,3-Difluorbenzol wird in einem inerten Lösungsmittel wie Diethylether, Tetrahydrofuran, Dimethoxyethan, tert-Butylmethylether oder Dioxan, Kohlenwasserstoffen wie Hexan, Heptan, Cyclohexan, Benzol oder

Toluol oder Gemischen dieser Lösungsmittel gegebenenfalls unter Zusatz eine Komplexierungsmittels wie Tetramethylethylendiamin (TMEDA) oder Hexamethylphosphorsäuretriamid mit Phenyllithium, Lithiumtetramethylpiperidin, n-, sek- oder tert-Butyllithium bei Temperaturen von -100 °C bis +50 °C vorzugsweise -78 °C bis 0 °C umgesetzt. Weitere Einzelheiten können der DOS 38 07 910 entnommen werden.

Die Lithium-2,3-difluorphenyl-Verbindungen werden bei -100 °C bis 0 °C vorzugsweise bei -50 °C mit den entsprechenden Elektrophilen umgesetzt. Geeignete Elektrophile sind Aldehyde, Ketone, Nitrile, Epoxide, Carbonsäure-Derivate wie Ester, Anhydride oder Halogenide, Halogenameisensäureester oder Kohlendioxid.

Zur Umsetzung mit aliphatischen oder aromatischen Halogen-Verbindungen werden die Lithium-2,3-difluorphenyl-Verbindungen transmetalliert und unter Übergangsmetallkatalyse gekoppelt. Besonders geeignet sind hierfür die Zink- (vgl. DE OS 36 32 410) oder die Titan-2,3-difluorphenyl-Verbindungen (vgl. DE OS 37 36 489).

Die Einführung heterocyclischer Strukturelemente kann einerseits dadurch erfolgen, daß man Vorstufen, die diese Strukturelemente bereits enthalten nach den bekannten Methoden zu den Verbindungen der Formel I umsetzt. Andererseits können aber auch in entsprechend strukturierten Vorstufen oder Unterstruktureinheiten der Verbindungen der Formel I nach an sich bekannten Methoden Heterocyclenrest erzeugt werden.

So können beispielsweise 2,5-disubstituierte 1,3,4-Thiadiazole durch Umsetzung von N,N'-Diacylhydrazinen mit üblichen Thiierungsreagenzien wie $P_4S_{10}$ oder Lawesson's Reagenz hergestellt werden. Die N,N'-Diacylhydrazine ihrerseits sind nach bekannten Methoden aus der entsprechenden Carbonsäuren zugänglich, wobei die Carbonsäuren mit einem 2,3-Difluor-1,4-phenylen-Strukturelement wie vorstehend beschrieben durch Umsetzung entsprechender metallisierter Vorstufen mit Kohlendioxid erhalten werden können.

Die 2,5-disubstituierten Pyrimidine können beispielsweise durch Umsetzung entsprechender Amidinhydrochloride (herstellbar aus den entsprechenden Carbonsäuren) mit Malondialdehydtetramethylacetalen nach an sich bekannten Methoden hergestellt werden. Die 2,5-disubstituierten Pyridine sind durch Kopplung von metallorganischen Zinkverbindungen mit entsprechenden Brompyridinderivaten entsprechend DE-OS 36 32 410 erhältlich. Die 2,5-disubstituierten Pyrazine sind erhältlich durch Kondensation von geeignet substituierten Ethylendiaminen mit Glyoxalderivaten, Oxidation der Dihydroverbindungen mit Luftsauerstoff oder anderen Oxidationsmitteln und Isolierung der gewünschten 2,5-disubstituierten Pyrazine aus dem entstandenen Gemisch der 2,5- und 2,6-Disubstitutionsprodukte. Die 3,6-disubstituierten Pyridazine sind zugänglich durch Umsetzung von 1,4-Diketonen (hergestellt z.B. nach Stetter durch thiazoliumsalzkatalysierte Addition eines Aldehyds an ein $\alpha,\beta$-ungesättigtes Keton) und anschließende Oxidation des Dihydropyridazins mit Luftsauerstoff oder anderen Oxidationsmitteln wie Kaliumnitrit oder Chromsäure in Eisessig.

Im folgenden wird die Synthese einiger besonders interesanter Hydroxy-Zwischenstufen beschrieben:

a) 5-Alkyl-2-(2,3-difluor-4-hydroxyphenyl)-pyridine sind erhältlich durch Umsetzung von 2,3-Difluor-4-benzyloxy-benzamidinhydrochlorid mit 2-Alkyl-3-ethoxyacroleinen bzw. mit in 2-Stellung alkylierten Malonaldehydtetraacetalen oder entsprechend substituierten vinylogen Formamidiniumsalzen (R.M. Wagner und CH. Jutz, Chem. Ber. 104 2975 (1971), indem man vorzugsweise die Komponenten in DMF (Dimethylformamid) erhitzt und anschließend die Schutzgruppe hydrogenolytisch abspaltet.

b) 5-Hydroxy-2-(2,3-difluor-4-alkylphenyl)-pyrimidine bzw. 5-Hydroxy-2(2,3-difluor-4-alkoxyphenyl)-pyrimidine sind erhältlich durch Kondensation von 4-Alkyl- bzw. 4-Alkoxy-2,4-difluorbenzamidinhydrochlorid mit 2-Benzyloxytrimethiniumperchlorat (A. Holy, Z. Arnold; Collection Czechoslov. Chem. Comm. 38 1371-1380 (1973), oder 2-Benzyloxy-3-dimethylaminoacrolein(H. Horstmann et al., Arzneimittelforsch. 11 682 (1961) und anschließender Hydrogenolyse der Benzylgruppe.

Nach einem Eintopfverfahren erhält man durch Zugabe eines Amidiniumsalzes in einem 12 Stunden bei 50° gerührten Reaktionsgemisch aus $POCl_3$,DMF und 2-Benzyloxyacetaldehyddiethylacetalund anschließender Zugabe von Triethylamin nach Abdestillieren des Triethylamins das entsprechende 5-Benzyloxypyrimidin.

c) 5-Hydroxy-2(2,3-difluor-4-alkylphenyl)pyridine bzw. 5-Hydroxy-2(2,3-difluor-4-alkoxyphenyl)pyridine sind erhältlich aus 2-Benzyloxytrimethiniumsalz durch Kondensation mit 4-Alkyl- oder 4-Alkoxy-2,3-difluoracetophenonen, Umsetzung mit $NH_3/NH_4Cl$ oder Ammoniumacetat.

Analog der Vorschriften von Ch. Jutz et al. (Liebigs Ann. Chem. 1975 874-900) und anschließende Hydrogenolyse oder aus 4-Alkyl- bzw. 4-Alkoxy-2,3-difluorphenylboronsäure durch Kopplung mit 5-Acetoxy-2-brompyridin (erhältlich aus 5-Hydroxy-2-brompyridin durch Veresterung) in Gegenwart eines Pd-Katalysators entsprechend den Arbeiten von Suzuki et al. (Synth. Commun. 11 513-19 (1981)).

d) 5-Alkoxy-2(2,3-difluor-4-hydroxyphenyl)pyridine sind erhältlich durch Kopplung von 2,3-Difluor-4-benzyloxyphenylboronsäure mit 5-Alkoxy-2-brompyridin entsprechend oben genannter Literatur und anschließende Hydrogenolyse.

e) 5-Alkyl-2(2,3-difluor-4-hydroxyphenyl)pyridine sind erhältlich durch Kopplung von 2-Brom-5-methylpyridin mit 2,3-Difluor-4-benzyloxyphenylboronsäure und einem Pd-Katalysator unter den bereits genannten Bedingungen, Kettenverlängerung der Methylgruppe durch Deprotonierung mit LDA als Base (-65 °C) und Alkylierung mit einem Alkylbromid und Hydrogenolyse.

f) 4-Alkoxy-2',3'-difluor-4'-hydroxybiphenyle bzw. 4-Alkyl-2',3'-difluor-4'-hydroxybiphenyle sind erhältlich wie oben beschrieben ausgehend von 1,2-Difluorbenzol (vgl. DOS 38 07 910).

g) 5-Alkyl-2(2,3-difluor-4-hydroxyphenyl)pyrimidine bzw. 5-Alkoxy-2(2,3-difluor-4-hydroxyphenyl)-pyrimidine sind herstellbar durch die übliche Kondensation von 2,3-Difluor-4-benzyloxybenzamidin mit 2-Alkylmalonaldehydtetraacetalen oder 2-Alkyl-3-ethoxyacroleinen bzw. 2,3-Dialkoxyacroleinen oder der entsprechenden Immoniumsalze oder Alkoxytrimethiniumsalzen und anschließender Hydrogenolyse.

Die besonders bevorzugten Verbindungen mit chiralen Fluoralkyloxy bzw. Fluoralkyl-Gruppen als Q* können wie folgt hergestellt werden:

Kommerziell erhältliche optisch aktive 1,2-Epoxide werden nach S. Brandange et al. (Acta Scand. B 37 - (1983) 141-145) mit HF/Pyridin zu den entsprechenden optisch aktiven 2-Fluor-1-alkanolen geöffnet. Diese können unter Standardbedingungen in die entsprechenden Tosylate und dann weiter nach Finkelstein in die Jodide überführt werden. Sowohl die Tosylate als auch die Jodide sind als Alkylierungsmittel geeignet, wobei Tosylate bevorzugt zur Veretherung von Phenolen eingesetzt werden. Eine weitere Möglichkeit zur Einführung optisch aktiver monofluorierter Seitenketten bildet die direkte Ringöffnung optisch aktiver 1,2-Epoxide durch Phenolate oder geeignete Kohlenstoffnucleophile. Diese Ringöffnungen verlaufen regiospezifisch am weniger substituierten C-Atom des Epoxids und liefern die entsprechenden optisch aktiven Alkohole, die dann unter Standardbedingungen mit DAST in die optisch aktiven Fluorverbindungen überführt werden können. Zur Ringöffnung geeignete Kohlenstoffnucleophile sind beispielsweise Grignard-Verbindungen, Acetylide, Enolate aber auch CH-azide Methylgruppen von Heterocyclen (z.B. Pyridin) oder geeignete substituierten Aromaten (z.B. p-Tolunitril).

So können die Verbindungen der Formel I oder zu deren Herstellung geeignete Vorstufen hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise -CH=CH-gruppen in Betracht, ferner z.B. freie oder veresterte Hydroxygruppen, aromatisch gebundene Halogenatome oder Carbonylgruppen. Bevorzugte Ausgangsstoffe für die Reduktion entsprechen der Formel I, können aber an Stelle einer $-CH_2CH_2-$Gruppe eine -CH=CH-Gruppe und/oder an Stelle einer $-CH_2-$Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) zu den entsprechenden Verbindungen der Formel I, die Alkylgruppen und/oder $-CH_2CH_2-$Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden; so entstehen z.B. aus 1-Cyancyclohexenderivaten die entsprechenden Cyclohexanderivate.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton,

Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedinungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20°.

Dioxanderivate bzw. Dithianderivate der Formel I werden zweckmäßig durch Reaktion eines entsprechenden Aldehyds (oder eines seiner reaktionsfähigen Derivate) mit einem entsprechenden 1,3-Diol bzw. einem entsprechenden 1,3-Dithiol (oder einem ihrer reaktionsfähigen Derivate) hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, alle können ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Zur Herstellung von Nitrilen der Formel I oder zur Herstellung von geeigneten Vorstufen können auch entsprechende Chlor- oder Bromverbindungen der Formel I oder geeignete Vorstufen mit einem Cyanid umgesetzt werden, zweckmäßig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen 20° und 200°.

Die optisch aktiven Verbindungen der Formel I erhält man durch den Einsatz entsprechender optisch aktiver Ausgangsmaterialien und/oder durch Trennung der optischen Antipoden mittels Chromatographie nach bekannten Methoden.

Die erfindungsgemäßen Phasen enthalten mindestens eine, vorzugsweise mindestens zwei Verbindungen der Formel I. Besonders bevorzugt sind erfindungsgemäße chirale getiltete smektische flüssigkristalline Phasen, deren achirale Basismischung mindestens eine andere Komponente mit negativer oder betragsmäßig kleiner positiver dielektrischer Anisotropie enthält. Die Chiralität beruht vorzugsweise teilweise oder vollständig auf chiralen Verbindungen der Formel I. Diese Phasen enthalten vorzugsweise eine oder zwei

chirale Verbindungen der Formel I. Es können jedoch auch achirale Verbindungen der Formel I (zum Beispiel in Form eines Racemates) eingesetzt werden, wobei dann die Chiralität der Phase durch andere optisch aktive Verbindungen hervorgerufen wird. Falls chirale Verbindungen der Formel I zum Einsatz kommen, eignen sich neben den reinen optischen Antipoden auch Gemische mit einem Enantiomerenüberschuß. Die oben erwähnten weiteren Komponente(n) der achiralen Basismischung können 1 bis 95 %, vorzugsweise 10 bis 90 %, der Mischung ausmachen. Als weitere Komponenten mit betragsmäßig kleiner positiver oder negativer dielektrischer Anisotropie eignen sich Verbindungen der Teilformeln IIa bis IIg:

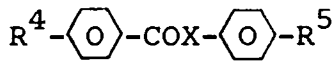

IIa

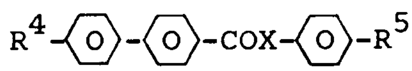

IIb

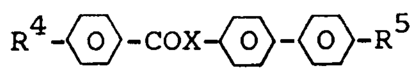

IIc

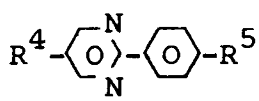

IId

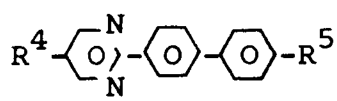

IIe

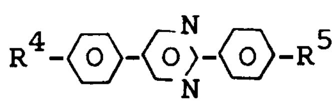

IIf

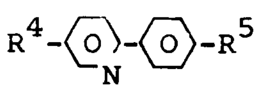

IIg

$R^4$ und $R^5$ sind jeweils vorzugsweise Alkyl, Alkoxy, Alkanoyloxy oder Alkoxycarbonyl mit jeweils 3 bis 12 C-Atomen. X ist vorzugsweise O. In den Verbindungen der Formeln IIa bis IIg kann auch eine 1,4-Phenylengruppe lateral durch Halogen, insbesondere bevorzugt durch Fluor, substituiert sein. Vorzugsweise ist einer der Gruppen $R^4$ und $R^5$ Alkyl und die andere Gruppe Alkoxy.

Besonders bevorzugt sind die Verbindungen der Teilformeln IIa bis IIg, worin $R^4$ und $R^5$ jeweils geradkettiges Alkyl oder Alkoxy mit jeweils 5 bis 10 C-Atomen bedeutet.

Ferner bevorzugt sind erfindungsgemäße Phasen, die neben Komponenten der Formeln IIa bis IIg noch mindestens eine Komponente mit deutlich negativer dielektrischer Anisotropie enthalten ($\Delta\epsilon \leqq -2$). Besonders geeignet sind hier Verbindungen der Formeln IIIa bis IIIc,

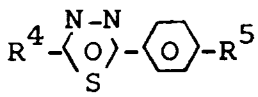

IIIa

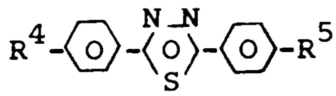

IIIb

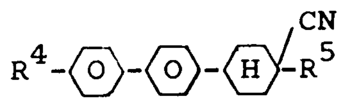

IIIc

worin $R^4$ und $R^5$ die bei den Formeln IIa bis IIg angegebenen allgemeinen und bevorzugten Bedeutungen haben. In den Verbindungen der Formeln IIIa, IIIb und IIIc kann auch eine 1,4-Phenylengruppe lateral durch Halogen, vorzugsweise Fluor, substituiert sein.

Die Verbindungen der Formel I umfassen insbesondere zweikernige und dreikernige Materialien. Von den zweikernigen, welche bevorzugt sind, sind diejenigen bevorzugt, worin $R^1$ n-Alkyl oder n-Alkoxy mit 7 bis 12, insbesondere 7 bis 9, C-Atome bedeutet.

Die erfindungsgemäßen Phasen enthalten vorzugsweise mindestens eine dreikernige Verbindung der Formel I. Diese Phasen zeichnen sich durch besonders hohe $S_C/S_A$-Umwandlungstemperaturen aus.

Die Verbindungen der Formel I eignen sich auch als Komponenten nematischer flüssigkristalliner Phasen, z.B. zur Vermeidung von reverse twist.

Diese erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl-oder Cyclohexylbenzoate, Cyclohexan-carbonsäurephenyl-oder cyclohexyl-ester, Phenylcyclohexane, Cyclohexylbiphenyle, Cyclohexylcyclohexane, Cyclohexylnaphthaline, 1,4-Bis-cyclohexylbenzole, 4,4′-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel I′ charakterisieren,

R′-L-G-E-R″ I″

worin L und E je ein carbo- oder heterocyclisches Ringsystem aus der aus 1,4-disubstituierten Benzol- und Cyclohexanringen, 4,4′-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

G -CH = CH- -N(O) = N-
-CH = CY- -CH = N(O)-
-C≡C- $-CH_2-CH_2-$
-CO-O- $-CH_2-O-$
-CO-S- $-CH_2-S-$
-CH = N- -COO-Phe-COO-
oder eine C-C-Einfachbindung,
Y Halogen, vorzugsweise Chlor, oder -CN, und

R′ und R″ Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R′ und R″ voneinander verschieden, wobei einer dieser Reste meist eine Alkyl- oder Alkoxygruppe ist. Aber auch andere Varianten der vorgesehenen Substituenten

sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95 %, einer oder mehrerer Verbindungen der Formel I. Weiterhin bevorzugt sind erfindungsgemäße flüssigkristalline Phasen, enthaltend 0,1-40, vorzugsweise 0,5-30 % einer oder mehrerer Verbindungen der Formel I.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst.Liq.Cryst. Band 24, Seiten 249 - 258 (1973)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. Fp. = Schmelzpunkt, Kp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

Beispiel 1

a) 323 g Butyraldehyd und 422 g Ethylcyanacetat werden in 750 ml Eisessig gelöst, 15 g Piperidin werden zugegeben und das Reaktionsgemisch gerührt. Dabei erwärmt sich das Reaktionsgemisch auf etwa 55 °C. Nach dem Abkühlen auf Raumtemperatur werden 20 g Pd-C-5 % Katalysator zugegeben, dann wird bei 30° und 2 bar $H_2$-Druck hydriert bis die berechnete Menge Wasserstoff aufgenommen worden ist (ca. 7 Stunden). Nach dem Abtrennen des Katalysators wird die Hydrierlösung am Rotationsverdampfer eingeengt, der Rückstand in Methyl-tertiärbutyl-Ether (MTB-Ether) aufgenommen und 2mal mit Wasser und anschließend mit 5%iger $NaHCO_3$-Lösung ausgeschüttelt. Die organische Phase wird getrocknet und eingedampft. Der Rückstand wird im Vakuum ($2 \times 10^{-2}$ mbar) destilliert (62 °C bis 78°). Man erhält 2-Butylcyanessigsäureethylester.

b) Unter Ausschluß von Sauerstoff und Luftfeuchtigkeit gibt man bei -60 °C zu 2000 ml einer 1,6 n Lösung von Butyllithium (BuLi) in Hexan und 2000 ml Tetrahydrofuran (THF) nacheinander 450 ml Diisopropylamin, 539 g 2-Butylcyanessigsäureethylester gelöst in 400 ml THF und dann 200 ml Methyljodid ebenfalls gelöst in 200 ml THF. Man läßt langsam auf Raumtemperatur erwärmen und arbeitet nachdem man 12 Stunden gerührt hat wie üblich auf. 256 g KOH-Plätzchen (85 %) werden in 3000 ml Methanol gelöst. Dann werden unter Eiskühlung 595 g 2-Butyl-2-methylcyanessigsäureethylester hinzugegeben und 3 Stunden unter weiterer Eiskühlung gerührt. Dann wird das Reaktionsgemisch mit konz. HCl angesäuert, mit Wasser verdünnt und mit MTB-Ether extrahiert. Die Etherphase wird mit 10%iger $NaHCO_3$-Lösung extrahiert und der Extrakt erneut unter Eiskühlung mit konz. HCl angesäuert. Das Produkt wird erneut mit MTB-Ether aus der wäßrigen Phase extrahiert, die organische Phase wird mehrmals mit gesättigter Kochsalzlösung gewaschen, anschließend getrocknet und eingeengt.

c) Racematspaltung

455 g 2-Butyl-2-Methylcyanessigsäure und 908 g Chinin werden in 2000 ml THF unter gelindem Erwärmen gelöst und 2 Stunden gerührt. Die auf Raumtemperatur abgekühlte Lösung gibt man dann langsam unter kräftigem Rühren in 6000 ml auf -50 °C abgekühltes Hexan, rührt 4 Stunden bei -40°/-50 °C und saugt dann den Niederschlag ab und trocknet ihn im Vakuum. Man kristallisiert das Salz mehrfach aus Hexan/THF um bis der Schmelzpunkt von 146,7 °C erreicht ist und setzt anschließend wie üblich die Säure frei.

$\alpha_D^{20}$ ($CHCl_3$): + 6,3

d) Zu 129 g 2-Butyl-2-Methylcyanessigsäure werden zusammen mit einer katalytischen Menge Dimethylformamid (DMF) auf 60 °C erwärmt, dann tropft man langsam 120 ml $SOCl_2$ zu. Es setzt eine stürmische Gasentwicklung ein. Wenn diese abgeklungen ist, erhitzt man 2 Stunden am Rückfluß, destilliert dann überschüssiges $SOCl_2$ im Vakkum ab und fraktioniert den Rückstand (4 x $10^{-2}$ mbar, 52-53 °C).

e) Man legt 0,1 mol 4′-Heptyloxy-2,3-difluor-4-hydroxybiphenyl (Herstellung: 0,1 mol 2,3-Difluor-4′-heptyloxybiphenyl (hergestellt durch Metallierung von 2,3-Difluorbenzol mit BuLi in Gegenwart von Tetramethylethylendiamin (TMEDA) in THF bei -80 °C und Umsetzung mit Heptyloxcyclohexanon sowie anschließende Dehydratisierung mit Toluol/Toluolsulfonsäure am Wasserabscheider und Aromatisierung mit DDQ) werden in 200 ml THF und 0,1 mol TMEDA gelöst, auf -78 °C abgekühlt und bei dieser Temperatur in 0,105 mol einer 1,6 n-Lösung von BuLi in Hexan metalliert und 3 Stunden gerührt. In der Zwischenzeit gibt man zu einer Lösung von 0,12 mol tert.-Butylhydroperoxid in 50 ml Ether innerhalb von 30 Minuten 70 ml einer 2 n-Lösung von Ethylmagnesiumbromid in Ether zu. Die so bereitete Lösung tropft man vorsichtig in die auf -78 °C gekühlte Lösung des metallierten 2,3-Difluor-4-heptyloxybiphenyls, läßt dann auf Raumtemperatur erwärmen und rührt dann nochmals 2 Stunden. Nach üblicher Aufarbeitung erhält man 4-Hydroxy-2,3-difluor-4′-heptyloxybiphenyl) und 0,11 mol Pyridin in Toluol vor und gibt dazu bei 50-60 °C eine Lösung von 2-Methyl-2-butylcyanessigsäurechlorid in etwas Toluol. Dann rührt man bei der angegebenen Temperatur 5 Stunden und arbeitet wie üblich auf. Man erhält optisch aktiven 2-Butyl-2-methylcyanessigsäure-(4′-heptyloxy-2,3-difluorbiphenyl-4-yl-ester).

Beispiel 2

Bei 0 °C gibt man zu einem Gemisch von 0,1 mol 4′-Heptyloxy-2,3-difluorbiphenylcarbonsäure (Herstellung: 0,1 mol 2,3-Difluor-4′-heptyloxybiphenyl und 0,1 mol TMEDA werden in 200 ml THF gelöst, auf -78 °C abgekühlt und bei dieser Temperatur mit 0,105 mol einer 1,6 n-Lösung von BuLi in Hexan umgesetzt. Man rührt 3 Stunden bei -78 °C und kippt dann das Reaktionsgemisch in einem Schwung auf 200 g gestoßenes Trockeneis. Nach üblicher Aufarbeitung erhält man 4′-Heptyloxy-2,3-difluor-biphenyl-4-carbonsäure), 0,1 mol optisch aktivem 2-Cyano-2-methylhexan-1-ol (herstellbar aus optisch aktivem 2-Methyl-2-butylcyanessigsäureethylester durch Reduktion mit $LiBH_4$) und einer katalytischen Menge 4-N,N′-Dimethylaminopyridin (DMAP) in 200 ml Methylenchlorid 0,1 mol DCC gelöst in Methylenchlorid. Anschließend läßt man 12 Stunden bei Raumtemperatur rühren. Man arbeitet wie üblich auf und reinigt das Produkt durch Kristallisation. Man erhält optisch aktiven 4′-Heptyloxy-2,3-difluorbiphenyl-4-carbonsäure-(2-cyan-2-methylhexylester).

Beispiel 3

Zu einer Lösung von 0,15 mol 4′-Heptyloxy-2,3-difluorbiphenyl-4-ol, 0,17 mol L(-)-Ethyllactat und 0,15 mol Triphenylphosphin in 400 ml THF gibt man 0,17 mol Azodicarbonsäurediethylester (DEAD) gelöst in THF. Dabei soll eine Reaktionstemperatur von 50 °C nicht überschritten werden. Man rührt 1 Stunde bei 50 °C und dann über Nacht bei Raumtemperatur. Anschließend destilliert man das Lösungsmittel ab, löst den Rückstand in heißem Toluol und läßt dann langsam abkühlen. Das ausgefallene Triphenylphosphinoxid wird abgesaugt, das Filtrat eingeengt und der Rückstand chromatographisch gereinigt. Man erhält 2-[4-(p-Heptyloxyphenyl)-2,3-difluorphenoxy]-propionsäureethylester.
Analog werden hergestellt: 2-[4-(5-Nonylpyrimidin-2-yl)-2,3-difluorphenoxy]-propionsäureethylester.

Beispiel 4

Zu 0,05 mol 4′-Heptyloxy-2,3-difluorbiphenyl-4-carbonsäure, 0,05 mol L(-)-Ethyllactat und einer katalytischen Menge DMAP in 70 ml Methylenchlorid gibt man bei 0 °C eine Lösung von 0,05 mol Dicyclohexylcarbodiimid (DCC) in Methylenchlorid. Anschließend rührt man 12 Stunden bei Raumtemperatur, dann saugt man den ausgefallenen Harnstoff ab und arbeitet wie üblich auf. Man erhält optisch aktiven 2-[4-(p-Heptyloxyphenyl)-2,3-difluorbenzoyloxy]-propionsäureethylester.

Beispiel 5

Optisch aktiver Milchsäurebenzylester wird mittels DCC und einer katalytischen Menge DMAP mit 4′-Heptyloxy-2,3-difluorbiphenyl-4-carbonsäure verestert und anschließend die Benzylgruppe hydrogenolytisch abgepalten. 0,01 mol der so gewonnenen Säure wir in 50 ml Benzol bei Raumtemperatur in Gegenwart

katalytischer Mengen von DMF mit 0,02 mol Oxalylchlorid in das entsprechende Säurechlorid überführt. Das Reaktionsgemisch wird im Vakuum eingedampft und der Rückstand in 30 ml Diglyme aufgenommen. Unter Kühlung und kräftigem Rühren werden tropfenweise 25 ml einer 30%igen wäßrigen Ammoniaklösung zugegeben. Man läßt 2 Stunden bei Raumtemperatur rühren, verdünnt mit Wasser, saugt den Niederschlag ab, wäscht ihn gründlich mit Wasser und trocknet ihn im Vakuum. Anschließend gibt man zu dem Niederschlag 40 ml DMF und 0,08 mol Thionylchlorid. Nachdem die Gasentwicklung nachgelassen hat, rührt man weitere 2 Stunden bei Raumtemperatur, dann hydrolysiert man vorsichtig und arbeitet wie üblich auf. Das Produkt wird chromatographisch gereinigt. Man erhält optisch aktiven 4′-Heptyloxy-2,3-difluorbiphenyl-4-carbonsäure-(1-cyanethylester).

Beispiel 6

Optisch aktiver Milchsäurebenzylester wird mittels Azodicarbonsäurediethylester (DEAD)-/Triphenylphosphin mit 4′-Heptyloxy-2,3-difluorbiphenyl-4-ol verethert und anschließend die Benzylgruppe hydrogenolytisch abgespalten. Die so gewonnene Säure wird wie üblich in das Nitril überführt (Oxalylchlorid, Ammoniak, Thionylchlorid). Man erhält optisch aktives 4′-Heptyloxy-2,3-difluor-4-(1-cyanethyoxy)-biphenyl.

Beispiel 7

Zu einem Gemisch aus 0,1 mol 4′-Heptyloxy-2,3-difluorbiphenyl-4-ol, 0,1 mol optisch aktiver 2-Chlor-3-methylbuttersäure (hergestellt aus Valin) und einer katalytischen Menge DMAP in 250 ml Methylenchlorid gibt man bei 0 °C eine Lösung von 0,1 mol DCC in Methylenchlorid. Anschließend läßt man 12 Stunden bei Raumtemperatur rühren, saugt dann den Niederschlag ab, arbeitet das Filtrat wie üblich auf und erhält 2-Chlor-3-methylbuttersäure-[4-(p-heptyloxyphenyl)-2,3-difluorphenylester].

Analog werden hergestellt durch Veresterung von analogen chiralen α-Halogencarbonsäuren mit 5-Alkyl-2-(2,3-difluor-4-hydroxyphenyl)-pyrimidinen:

2-Fluoroctansäure-4-(5-heptylpyrimidin-2-yl)-2,3-difluorphenylester , F 39°

2-Chlor-3-methylbuttersäure-4-(5-heptylpyrimidin-2-yl)-2,3-difluorphenylester , F 65°

Beispiel 8

Unter Ausschluß von Feuchtigkeit gibt man zu einem Gemisch von 0,1 mol Pentansäure, 0,1 mol optisch aktivem 4′-Heptyloxy-2,3-difluor-4(2-hydroxypropyl)biphenyl (herstellbar durch übliche Metallierung von 4′-Heptyloxy-2,3-difluorbiphenyl und Umsetzung mit optisch aktivem Propylenoxid) und einer katalytischen Menge DMAP in 200 ml Methylenchlorid bei 0 °C 0,1 mol DCC gelöst in Methylenchlorid. Nach der Zugabe läßt man 12 Stunden bei Raumtemperatur rühren, saugt den Niederschlag ab und arbeitet wie üblich auf. Das Produkt wird chromatographisch gereinigt. Man erhält optisch aktives 4′-Heptyloxy-2,3-difluor-4-(2-valeroyloxypropyl)-biphenyl.

Beispiel 9

Unter Ausschluß von Feuchtigkeit gibt man zu einem Gemisch von 0,1 mol Pentansäure, 0,1 mol optisch aktivem 2(4′-Heptyloxy-2,3-difluorbiphenyl-4-oxy)propan-1-ol (hergestellt durch Reduktion des entsprechenden Milchsäureethylesters mit $LiBH_4$) und einer katalytischen Menge DMAP in 250 ml Methylenchlorid bei 0 °C 0,1 mol DCC gelöst in Methylenchlorid. Anschließend läßt man bei Raumtemperatur 12 Stunden rühren, saugt den Niederschlag ab, arbeitet das Filtrat wie üblich auf und erhält optisch aktives 4′-Heptyloxy-2,3-difluor-4-(1-valeroyloxy-2-propyloxy)biphenyl.

Beispiel 10

Optisch aktiver S-2-Hydroxy-3-methylbuttersäurebenzylester (hergestellt aus dem Cäsiumsalz der L-α-Hydroxyisovaleriansäure durch Umsetzung mit Benzylbromid in DMF) und 4′-Heptyloxy-2′,3′-difluorbiphenyl-4-carbonsäure (hergestellt durch Metallierung von Heptyloxy-2,3-difluorbenzol mit BuLi und TMEDA in THF bei -70 °C, Ummetallisierung mit Chlortriisopropylorthotitanat und anschließende Umsetzung mit 4-Cyclohexanoncarbonsäureethylester, Dehydratisierung, Aromatisierung mit DDQ und Verseifung mit ethanolischer KOH bei 5 °C) wird mittels DCC und einer katalytischen Menge DMAP verestert und anschließend die Benzylgruppe hydrogenolytisch abgespalten. 0,01 mol der so gewonnen Säure wird in 75 ml Toluol bei 25

°C in Gegenwart einer katalytischen Menge DMF mit 0,02 mol Oxalylchlorid in das Säurechlorid überführt. Das Reaktionsgemisch wird im Vakuum eingedampft und der Rückstand in wenig Diglyme aufgenommen und unter Kühlung und kräftigem Rühren tropfenweise mit 30 ml einer 30%igen wäßrigen Ammoniaklösung versetzt. Man läßt 2 Stunden bei Raumtemperatur rühren, verdünnt mit Wasser, saugt den Niederschlag ab, wäscht ihn mit Wasser ammoniakfrei und trocknet ihn im Vakuum. Anschließend gibt man 40 ml DMF und tropfenweise 0,1 mol Thionylchlorid zu. Man rührt zwei 2 Stunden bei Raumtemperatur und gibt dann das Reaktionsgemisch auf Eis/Wasser. Es wird wie üblich aufgearbeitet und das Produkt chromatographisch gereinigt. Man erhält 4′-Heptyloxy-2′,3′-difluorbiphenyl-4-carbonsäure-(1-cyan-2-methylpropylester).

Analog werden hergestellt:

4′-Heptyloxy-2,2′,3′-trifluorbiphenyl-4-carbonsäure-(1-cyan-2-methylpropylester)
4′-Heptyloxy-3,2′,3′-trifluorbiphenyl-4-carbonsäure-(1-cyan-2-methylpropylester)
4′-Heptyloxy-2,3,3′-trifluorbiphenyl-4-carbonsäure-(1-cyan-2-methylpropylester)
4′-Heptyloxy-2,3,2′-trifluorbiphenyl-4-carbonsäure-(1-cyan-2-methylpropylester)

Beispiel 11

2,3-Difluorhydrochinonmonobenzylether (hergestellt aus 2,3-Difluorphenol durch Elbs-Reaktion, Benzylierung des als Zwischenprodukt erhaltenen Sulfates und saure Spaltung des Sulfates) wird mit DEAD/$PPh_3$ und optisch aktivem Milchsäureethylester umgesetzt. Anschließend wird die Schutzgruppe hydrogenolytisch abgespalten. Unter Feuchtigkeitsausschluß werden in Methylenchlorid 0,01 mol der so erhaltenen Verbindung und 0,01 mol 4′-Heptyloxybiphenyl-4-carbonsäure in Gegenwart einer katalytischen Menge DMAP tropfenweise bei 0 °C mit 0,01 mol DCC gelöst in Methylenchlorid umgesetzt. Man läßt 12 Stunden bei Raumtemperatur rühren, entfernt den ausgefallenen Dicyclohexylharnstoff durch Filtration und arbeitet das Filtrat wie üblich auf. Das Produkt wird chromatographisch gereinigt. Man erhält 2-[2,3-Difluor-4-(p-(p-heptyloxyphenyl)-benzoyloxy)-phenoxy]-propionsäureethylester.

Beispiel 12

0,08 mol 5-Heptyl-2-(2,3-difluor-4-hydroxyphenyl)-pyrimidin werden zusammen mit 11,6 g $K_2CO_3$ und 25,6 g optisch aktivem 2-Fluor-1-Octyltosylat in 70 ml Methylethylketon unter Schutzgasatmosphäre 12 Stunden am Rückfluß erhitzt. Dann wird das Reaktionsgemisch hydolysiert, das Produkt wie üblich aufgearbeitet und chromatographisch gereinigt. Man erhält optisch aktives 5-n-Heptyl-2-[2,3-difluor-4-(2-fluor-octyloxy)-phenyl)-pyrimidin, F 56° C.

In völlig analoger Weise können auch alle anderen geeigneten Phenole bzw. Hydroxyverbindungen mit chiralen 2-Fluor-1-alkyltosylaten umgesetzt werden.

Beispiel 13

0,04 mol 5-(3-hydroxynonyl)-2-(2,3-difluor-4-octyl-oxyphenyl)-pyridin (optisch aktiv) [herstellbar aus 5-Methyl-2-(2,3-difluor-4-octyloxyphenyl)-pyridin durch Umsetzung mit LDA bei -70 °C und Zugabe von optisch aktivem 1,2-Epoxyoctan] werden unter Feuchtigkeitsausschluß bei -30 °C in $CH_2Cl_2$ gelöst. Zu dieser Lösung tropft man langsam unter Kühlung 8 ml DAST gelöst in 15 ml $CH_2Cl_2$. Dann wird das Reaktionsgemisch 12 Stunden bei Raumtemperatur gerührt und anschließend mit Eiswasser hydrolysiert. Man arbeit wie üblich auf und erhält optisch aktives 5-(3-fluornonyl)-2-(2,3-difluor-4-octyloxyphenyl)-pyridin.

Beispiel 14

0,08 mol 2,3-Difluor-4-(3-fluornonyl)-benzamidinhydrochlorid (optisch aktiv) [erhältlich aus 2,3-Difluor-4-methylbenzonitril durch Umsetzung mit LDA bei -70 °C und Zugabe von chiralem 1,2-Epoxyoctan, Umsetzung des entstandenen Alkohols mit DAST und Überführung der Nitrilgruppe ins Amidin] werden zusammen mit 0,1 mol 2-Heptyl-3-ethoxyacrolein in DMF 24 Stunden erhitzt. Nach üblicher Aufarbeitung erhält man optisch aktives 5-n-Heptyl-2-[2,3-difluor-4-(3-fluor-n-nonyl)-phenyl]-pyrimidin.

In analoger Weise erhält man aus den 2,3-Dialkoxyacroleinen die entsprechenden 5-n-Alkoxy-pyrimidine.

Beispiel 15

0,05 mol 2,3-Difluor-4-hydroxybenzoesäuremethylester, 0,05 mol (S)-2-Fluoroctanol-1 und 0,06 mol Triphenylphosphin werden in 125 ml Tetrahydrofuran gelöst und unter Rühren und Eiskühlung 0,06 mol Diethylazodicarboxylat zugetropft. Man läßt auf Raumtemperatur kommen und rührt noch 8 Stunden nach. Danach wird das Lösungsmittel abdestilliert und das Methyl-2,3-difluor-4-(2-fluoroctyloxy)-benzoat durch Säulenchromatographie gereinigt. Durch Verseifung mit wäßrig/alkoholischer Kalilauge erhält man daraus die 2,3-Difluor-4-(2-fluoroctyloxy)-benzoesäure.

0,01 mol dieser Säure, 0,001 mol 4-Dimethylaminopyridin und 0,01 mol 4-n-Octylphenol werden in 15 ml Dichlormethan vorgelegt, bei 10° unter Rühren eine Lösung von 0,01 mol Dicyclohexylcarbodiimid zugetropft und anschließend 15 Stunden bei Raumtemperatur nachgerührt. Man filtriert über Kieselgel ab, verdampft das Lösungsmittel und erhält als Ruckstand (S)-(4-n-Octylphenyl)-2,3-difluor-4-(2-fluoroctyloxy)-benzoat.

Beispiel 16

Analog Beispiel 15 erhält man (S)-[trans,trans-4-(4-n-Pentylcyclohexyl)cyclohexyl]-2,3-difluor-4-(2-fluoroctyloxy)-benzoat, wenn man trans,trans-4-(4-Pentylcyclohexyl)-cyclohexanol als Alkoholkomponente in die vorstehend beschriebene Veresterung einsetzt.

Beispiel 17

0,01 mol (S)-2,3-Difluor-4-(2-fluoroctyloxy)-benzoylchlorid (hergestellt aus der in Beispiel 15 beschriebenen Säure durch Erhitzen mit Thionylchlorid), 0,01 mol 4-trans-4-n-Pentylcyclohexyl)phenol und 0,01 mol Pyridin werden in 20 ml Toluol 2 Stunden unter Rückfluß erhitzt. Nach dem Abkühlen saugt man das Pyridinhydrochlorid ab, verdampft das Toluol und kristallisiert das zurückbleibende (S)-[4-(trans-4-n-Pentylcyclohexyl)phenyl]-2,3-difluor-4-(2-fluoroctyloxy)benzoat aus Ethanol um.

Beispiel 18

0,05 mol 2-(2,3-Difluor-4-hydroxyphenyl)-5-n-nonylpyrimidin, 0,5 mol Kaliumcarbonat und 40 ml Dimethylformamid werden unter Rühren zum Rückfluß erhitzt. Man tropft innerhalb von 5 Minuten 0,05 mol (R)-1,2-epoxyoctan zu und läßt 16 Stunden nachreagieren. Anschließend wird das Reaktionsgemisch auf 100g Eis gegossen und das ausgefallene (R)-2-[2,3-Difluor-4-(2-hydroxyoctyloxy)-phenyl]-5-n-nonylpyrimidin abgesaugt, mit Wasser gewaschen und aus Aceton umkristallisiert.

0,03 mol dieser Verbindung werden in 40 ml Dichlormethan gelöst und bei -70° unter Stickstoff zu einer gerührten Lösung von 0,045 mol Diethylaminoschwefeltrifluorid in 15 ml Dichlormethan getropft. Man läßt langsam auf Raumtemperatur kommen und über Nacht nachreagieren. Danach wird das Reaktionsgemisch unter Kühlung mit Wasser versetzt, 30 Minuten gerührt, die organische Phase abgetrennt, mit Wasser gewaschen und getrocknet. Nach dem Abdestillieren des Lösungsmittels wird das (S)-2-[2,3-Difluor-4-(2-fluoroctyloxy)-phenyl]-5-n-nonylpyrimidin aus Ethanol umkristallisiert.

Beispiele 19-21

Analog Beispiel 18 erhält man aus den entsprechenden Phenolen durch Umsetzung mit (R)-1,2-Epoxyheptan und anschließende Fluorierung mit Diethylaminoschwefeltrifluorid die Verbindungen
(S)-2,3-Difluor-4-(2-fluorheptyloxy)-4′-(5-n-heptylpyrimidin-2-yl)-biphenyl,
(S)-2-[2,3-Difluor-4-(2-fluorheptyloxy)-phenyl]-5-n-nonylpyridin und
(S)-2-[2,3-Difluor-4-(2-fluorheptyloxy)-phenyl]-5-(4-n-heptylphenyl)-pyridin.

Beispiel 22

0,02 mol 2,3-Difluor-4-hydroxy-4′-pentyl-p-terphenyl, 0,02 mol Kaliumcarbonat und 0,2 mol (S)-2-Fluoroctyl-p-toluolsulfonat (hergestellt aus (S)-2-Fluoroctanol-1 und Toluolsulfochlorid in Gegenwart von Pyridin) werden in 25 ml Dimethylformamid 15 Stunden bei 60° gerührt. Man saugt von den ungelösten Salzen ab und versetzt das Filtrat mit Wasser. Durch extraktive Aufarbeitung und anschließende Säulenchromatographie erhält man das reine (S)-2,3-Difluor-4-(2-fluoroctyloxy)-4'-pentyl-p-terphenyl.

Beispiel 23

Analog Beispiel 22 erhält man aus 2,3-Difluor-4-hydroxy-4'-(trans-4-petnylcyclohexyl)-biphenyl durch Veretherung mit (S)-2-Fluornonyl-p-toluolsulfonat (S)-2,3-Difluor-4-(2-fluornonyloxy)-4'-(trans-4-pentylcyclohexyl)-biphenyl.

Die folgenden Beispiele betreffen erfindungsgemäße flüssigkristalline Phasen:

Beispiel A bis L

Eine achirale $S_C$-Basismischung bestehend aus

4,4 % 2-p-Heptyloxyphenyl-5-heptylpyrimidin,
4,4 % 2-p-Octyloxyphenyl-5-heptylpyrimidin,
4,4 % 2-p-Nonyloxyphenyl-5-heptylpyrimidin,
7,8 % 2-(2,3-Difluor-4-octyloxyphenyl)-5-heptylpyrimidin,
7,8 % 2-p-Hexyloxyphenyl-5-nonylpyrimidin,
25,6 % 2-p-Nonyloxyphenyl-5-nonylpyrimidin,
10,0 % 2-(2,3-Difluor-4-nonyloxyphenyl)-5-nonylpyrimidin,
8,9 % 2-(p-Hexyloxyphenyl)-5-(p-heptylphenyl)-1,3,4-thiadiazol,
11,1 % r-1-Cyan-cis-4-(4'-octyloxybiphenyl-4-yl)-1-octylcyclohexan,
8,9 % 2-(4′-Hexyloxy-2,3-difluorbiphenyl-4-yl)-5-heptylpyrimidin und
6,7 % 2-(2,3-Difluor-4-pentyloxyphenyl)-5-(p-heptylphenyl)-1,3,4,-thiadiazol

wird mit jeweils 10 % der folgenden Dotierstoffe A bis L versetzt. Die Phasenübergangstemperaturen und die Werte der Spontanpolarisation bei Raumtemperatur sind in der folgenden Tabelle zusammengefaßt:

| Dotierstoff | $S_C^*$ | $S_A$ | Ch | I | $P_S$ ($nC/cm^2$) |
|---|---|---|---|---|---|
| A | 65 | 70 | 81 | | 20 |
| B | 62 | - | 78 | | 11 |
| C | 56 | 61 | 74 | | 21 |
| D | 67 | 72 | 84 | | 14 |
| E | 62 | 64 | 75 | | 10 |
| F | 70 | 73 | 87 | | 17 |
| G | 63 | 67 | 78 | | 12 |
| H | 64 | - | 79 | | 12 |
| I | 51 | 58 | 73 | | 11 |
| J | 60 | 65 | 76 | | 10 |
| K | 58 | 62 | 77 | | 25 |
| L | 72 | 78 | 88 | | 18 |

Art der Dotierstoffe

A: 2-Butyl-2-methylcyanessigsäure-(4'-heptyloxy-2,3-difluorbiphenyl-4-ylester)

B: 2-[4-(p-Heptyloxyphenyl)-2,3-difluorphenoxy]-propionsäureethylester

C: 4'-Heptyloxy-2,3-difluorbiphenyl-4-carbonsäure-(1-cyanethylester)

D: 2-Chlor-3-methylbuttersäure-[4-(p-Heptyloxyphenyl)-2,3-difluorphenylester]

E: 4'-Heptyloxy-2,3-difluor-4-(1-valeroyloxy-2-propyloxy)-biphenyl

F: 2-[4-(4'-Nonyloxy-2',3'-difluorbiphenyl-4-yl)-phenoxy]-propionsäureethylester

G: 4'-Heptyloxy-2,3-difluorbiphenyl-4-carbonsäure-(2-cyan-2-methylhexylester)

H: 2-[4-(p-Heptyloxyphenyl)-2,3-difluorbenzoyloxy]-propionsäureethylester

I: 4'-Heptyloxy-2,3-difluor-4-(1-cyanethoxy)-biphenyl

J: 4'-Heptyloxy-2,3-difluor-4-(2-valeroyloxypropyl)-biphenyl

K: 4'-Heptyloxy-2',3'-difluorbiphenyl-4-carbonsäure-(1-cyan-2-methylpropylester)

L: 2-[2,3-Difluor-4(p-(p-heptyloxyphenyl)-benzoyloxy)-phenoxy]-propionsäureethylester

## Patentansprüche

**1.** Chirale Derivate des 1,2-Difluorbenzols der Formel I,

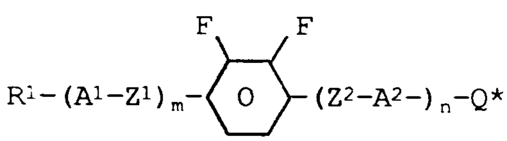

I

worin

$R^1$ H, F, Cl, Br, CN, eine Alkyl- oder Perfluoralkyl-Gruppe mit jeweils 1 bis 12 C-Atomen, worin auch eine oder zwei nicht benachbarte $CH_2$- bzw. $CF_2$-Gruppen durch O-Atome und/oder -CO-Gruppen und/oder -CO-O-Gruppen und/oder -CH=CH-Gruppen und/oder -CHHalogen und/oder -CO-O-CHCN-Gruppen ersetzt sein können, oder einen optisch aktiven Rest der Formel

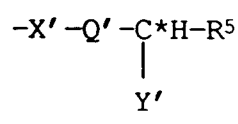

worin

X' -CO-O-, -O-CO-, -O-CO-O, -CO-, -O-, -S-, -CH=CH-, -CH=CH-COO- oder eine Einfachbindung,

Q' Alkylen mit 1 bis 5 C-Atomen, worin auch eine nicht mit X' verknüpfte $CH_2$-Gruppe durch -O-, -CO-, -O-CO- oder -CH=CH-ersetzt sein kann, oder eine Einfachbindung,

Y' CN, Halogen oder Methyl, und

$R^5$ eine von Y' verschiedene Alkylgruppe mit 1 bis 15 C-Atomen, worin auch eine oder zwei nicht benachbarte $CH_2$-Gruppen durch -O-, -CO-, -O-CO-, -CO-O- und/oder -CH=CH- ersetzt sein können,

$A^1$ und $A^2$ jeweils unabhängig voneinander unsubstituiertes oder durch ein oder zwei F- und/oder Cl-Atome und/oder $CH_3$-Gruppen und/oder CN-Gruppen substituiertes 1,4-Phenylen,

| | |
|---|---|
| | worin auch eine oder zwei CH-Gruppen durch N ersetzt sein können, 1,4-Cyclohexylen, worin auch eine oder zwei nicht benachbarte $CH_2$-Gruppen durch O-Atome und/oder S-Atome ersetzt sein können, Piperidin-1,4-diyl, 1,4-Bicyclo(2,2,2)octylen, 1,3,4-Thiadiazol-2,5-diyl, Naphthalin-2,6-diyl, Decahydronaphthalin-2,6-diyl- oder 1,2,3,4-Tetrahydronaphthalin-2,6-diyl, |
| $Z^1$ und $Z^2$ | jeweils -CO-O-, -O-CO-, $-CH_2CH_2-$, $-OCH_2-$, $-CH_2O-$, -C≡C- oder eine Einfachbindung, |
| m | 0, 1 oder 2, |
| n | 0, 1 oder 2, |
| (m + n) | 1 oder 2, und |
| Q* | einen Chiralität induzierenden organischen Rest mit einem asymmetrischen Kohlenstoffatom bedeutet. |

**2.** Chirale Derivate des 1,2-Difluorbenzols der Formel I nach Anspruch 1, dadurch gekennzeichnet, daß Q* einen Rest der Formel

$Q^1$-C*R°X-$Q^2$-$R^2$

bedeutet, worin

| | |
|---|---|
| $Q^1$ und $Q^2$ | jeweils unabhängig voneinander Alkylen mit 2 bis 4 C-Atomen, worin auch eine $CH_2$-Gruppe durch -O-, -S-, -CO-, -O-CO-, -CO-O-, -S-CO-, -CO-S-, -CH=CH-COO-, -CH=CH-, -CHHalogen und/oder -CHCN- ersetzt sein kann, oder eine Einfachbindung, |
| X | Halogen, CN oder $CH_3$, |
| R° | H oder eine von X und -$Q^2$-$R^2$ verschiedene Alkylgruppe mit 1 bis 10 C-Atomen, |
| $R^2$ | eine Alkylgruppe mit zwei bis 10 C-Atomen, und |
| C* | ein mit vier verschiedenen Substituenten verknüpftes Kohlenstoffatom bedeutet. |

**3.** Chirale Derivate des 1,2-Difluorbenzols der Formel I nach Anspruch 1, dadurch gekennzeichnet, daß Q* einen Rest der Formel

$Q^1$-C*R°X-$Q^2$-$R^2$

bedeutet, worin

| | |
|---|---|
| $Q^1$ und $Q^2$ | jeweils unabhängig voneinander Alkylen mit 1 bis 2 C-Atomen, -O-, -O-CO-, -CO-O-, $-CH_2-O-$, $-O-CH_2-$ oder eine Einfachbindung bedeuten, und |
| R°, $R^2$, C* und X | die in Anspruch 2 angegebene Bedeutung besitzen. |

**4.** Verbindungen nach einem der Ansprüche 1 bis 3, worin $-(A^1-Z^1)_m-PheF_2-(Z^2-A^2)_n-$ eine Gruppe der folgenden Formeln 1 bis 15 oder deren Spiegelbild bedeutet:

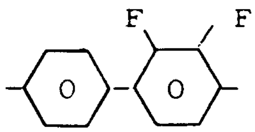

1

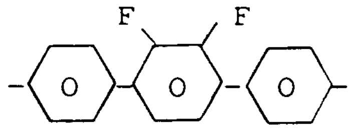

2

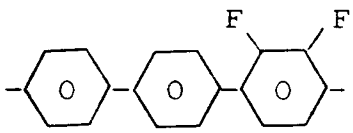

3

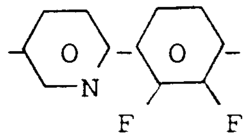

4

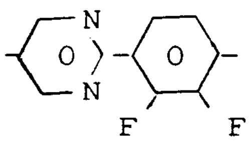

5

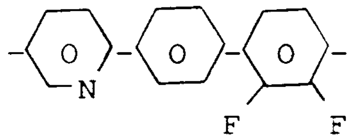

6

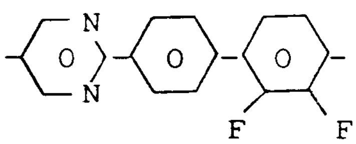

7

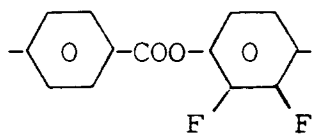

8

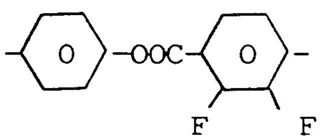

9

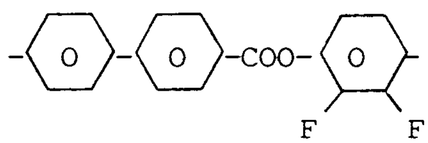

10

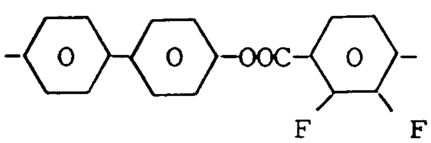

11

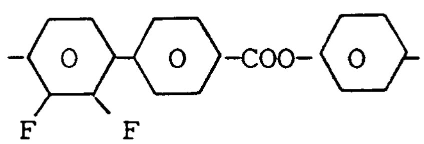

12

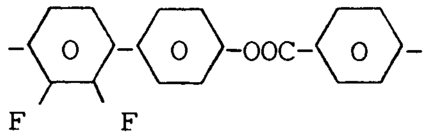

13

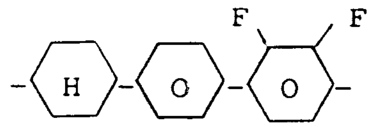

14

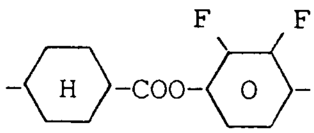

15

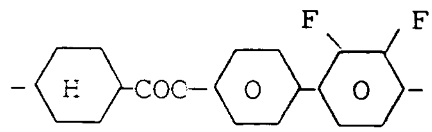

16

**5.** Verbindungen nach einem der Ansprüche 1 bis 4, ausgewählt aus den Formeln A-M

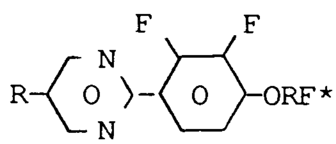

A

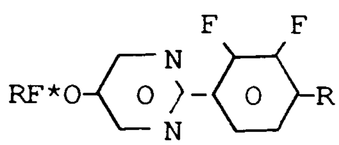

B

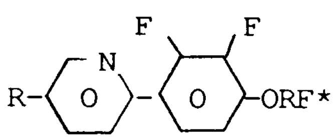

C

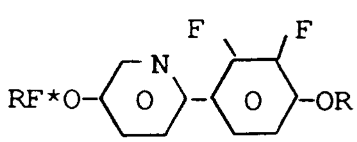

D

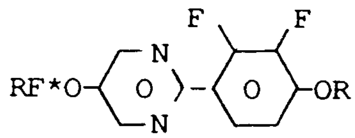

E

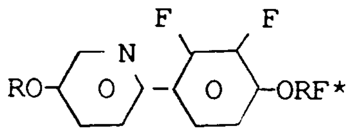

F

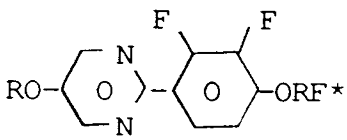

G

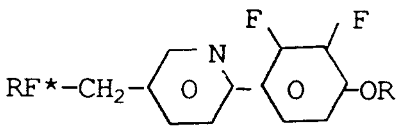

H

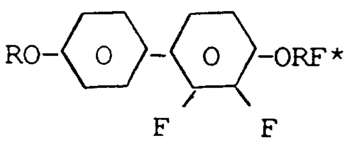

I

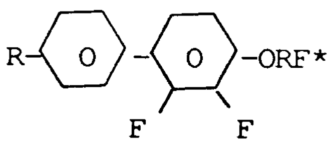

K

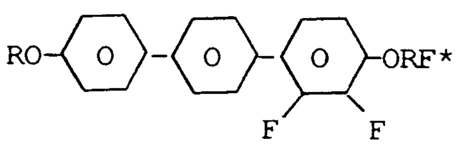

L

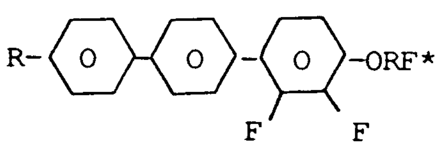

M

worin RF* geradkettiges oder einfach verzweigtes Alkyl mit 3 bis 12 C-Atomen, worin eine $CH_2$-Gruppe durch -C*HF-ersetzt ist und C* ein asymmetrisches C-Atom ist, vorzugsweise

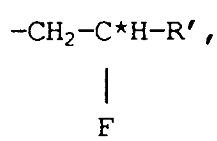

worin R' geradkettiges oder einfach verzweigtes Alkyl mit vorzugsweise 2 bis 10, insbesondere 3 bis 10 C-Atomen ist, und R eine der Bedeutungen $R^1$ aufweist.

**6.** Verbindungen nach einem der Ansprüche 1 bis 5 der Formel A'

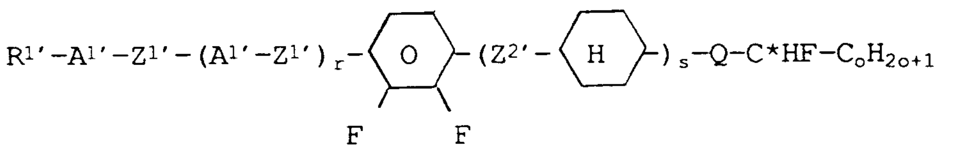

A'

worin

| | |
|---|---|
| $R^{1'}$ | einen unsubstituierten, einen einfach durch -CN oder einen mindestens einfach durch Fluor oder Chlor substituierten Alkyl- oder Alkenylrest mit bis zu 12 C-Atomen, wobei in diesen Resten auch eine $CH_2$-Gruppe durch -O-, -CO- oder -CO-O- ersetzt sein kann, |
| $A^{1'}$ | jeweils unabhängig voneinander einen unsubstituierten oder durch ein oder zwei Fluoratome substituierten 1,4-Phenylen-Rest, Pyridin-2,5-diyl-Rest, Pyrimidin-2,5-diyl-Rest, Pyrazin-2,5-diyl-Rest, Pyridazin-3,6-diyl-Rest, 1,3,4-Thiadiazol-2,5-diyl-Rest, trans-1,4-Cyclohexylen-Rest, worin auch eine oder zwei nicht benachbarte $CH_2$-Gruppen durch -O-und/oder -S- ersetzt sein können, 1,4-Bicyclo(2.2.2)octylen-Rest oder einen Piperidin-1,4-diyl-Rest, |
| $Z^{1'}$ und $Z^{2'}$ | jeweils unabhängig voneinander -CO-O-, -O-CO-, $-CH_2O-$, $-OCH_2-$, $-CH_2CH_2-$, -C≡C- oder eine Einfachbindung |
| Q | $-OCH_2-$, $-COOCH_2-$ oder $-CH_2OCH_2-$ |
| o | 1 bis 12, |

und einer der beiden Werte

r und s 0 und der andere 0 oder 1 bedeutet.

**7.** Chirale getiltete smektische flüssigkristalline Phase mit mindestens zwei flüssigkristallinen Komponenten, dadurch gekennzeichnet, daß sie mindestens eine Verbindung der Formel I nach Anspruch 1 enthält.

**8.** Phase nach Anspruch 7, dadurch gekennzeichnet, daß sie mindestens eine Verbindung der Teilformeln IIa bis IIg enthält:

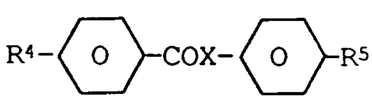

IIa

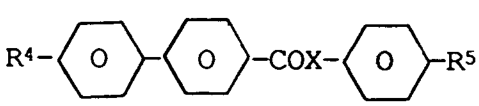

IIb

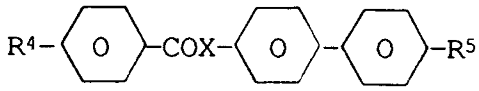

IIc

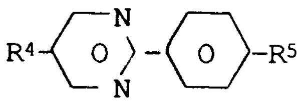

IId

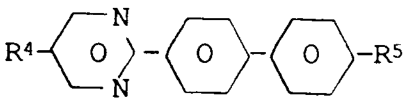

IIe

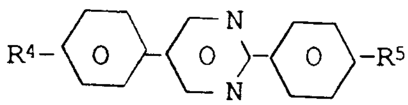

IIf

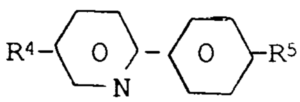

IIg

worin $R^4$ und $R^5$ jeweils Alkyl, Alkoxy, Alkanoyloxy oder Alkoxycarbonyl mit jeweils 3 bis 12 C-Atomen, und X 0 bedeutet, wobei auch eine 1,4-Phenylengruppe lateral durch Halogen, insbesondere bevorzugt durch Fluor, substituiert sein kann.

**9.** Phase nach Anspruch 8, dadurch gekennzeichnet, daß sie neben Komponenten der Formeln IIa bis IIg noch mindestens eine Verbindung der Formeln IIIa bis IIIc enthält:

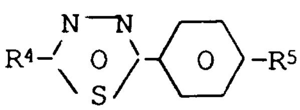

IIIa

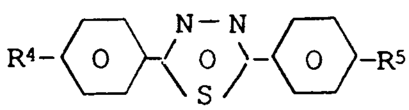

IIIb

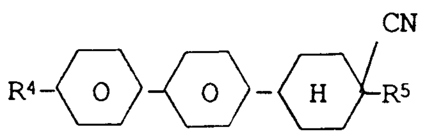

IIIc

worin $R^4$ und $R^5$ die bei den Formeln IIa bis IIg angegebene Bedeutung haben.

**10.** Verwendung der Verbindungen der Formel I nach Anspruch 1 als Komponenten flüssigkristalliner Phasen.

**11.** Ferroelektrisches elektrooptisches Anzeigeelement, dadurch gekennzeichnet, daß es als Dielektrikum eine Phase nach Anspurch 7 enthält.

**Claims**

**1.** Chiral 1,2-difluorobenzene derivatives of the formula I

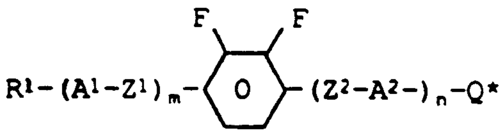

I

in which

| | |
|---|---|
| $R^1$ | is H, F, Cl, Br, CN, an alkyl or perfluoroalkyl group each having 1 to 12 C atoms and in which, in addition, one or two non-adjacent $CH_2$ or $CF_2$ groups may be replaced by O atoms and/or -CO-groups and/or -CO-O- groups and/or -CH=CH-groups and/or -CHhalogen- and/or -CO-O-CHCN-groups, or an optically active radical of the formula |

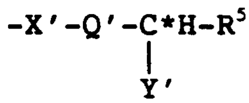

in which

| | |
|---|---|
| X' | is -CO-O-, -O-CO-, -O-CO-O, -CO-, -O-, -S-, -CH=CH-, -CH=CH-COO- or a single bond, |
| Q' | is alkylene having 1 to 5 C atoms and in which, in addition, one $CH_2$ group which is not linked to X' may be replaced by -O-, -CO-, -O-CO- or -CH=CH-, or a single bond, |
| Y' | is CN, halogen or methyl, and |
| $R^5$ | is an alkyl group different from Y' and having 1 to 15 C atoms, and in which, in addition, one or two non-adjacent $CH_2$ groups may be replaced by -O-, -CO-, -O-CO-, -CO-O-, and/or -CH=CH-, |
| $A^1$ and $A^2$, | in each case independently of one another, are 1,4-phenylene which is unsubstituted or substituted by one or two F and/or Cl atoms and/or $CH_3$ groups and/or CN groups and in which, in addition, one or two CH groups may be replaced by N, or are 1,4-cyclohexylene in which, in addition, one or two non-adjacent $CH_2$ groups may be replaced by O atoms and/or S atoms, or are piperidine-1,4-diyl, 1,4-bicyclo(2,2,2)-octylene, 1,3,4-thiadiazole-2,5-diyl, naphthalene-2,6-diyl, decahydronaphthalene-2,6-diyl or 1,2,3,4-tetrahydronaphthalene-2,6-diyl, |
| $Z^1$ and $Z^2$ | are each -CO-O-, -O-CO-, $-CH_2CH_2-$, $-OCH_2-$, $-CH_2O-$, -C=C- or a single bond, |
| m | is 0, 1 or 2, |
| n | is 0, 1 or 2, |
| (m + n) | is 1 or 2, and |
| Q* | is a chirality-inducing organic radical having an asymmetric carbon atom. |

**2.** Chiral 1,2-difluorobenzene derivatives of the formula I according to Claim 1, characterized in that Q* is a radical of the formula

$-Q^1-C^*R^oX-Q^2-R^2$

in which

| | |
|---|---|
| $Q^1$ and $Q^2$, | in each case independently of one another, are alkylene having 2 to 4 C atoms in which, in addition, one $CH_2$ group may be replaced by -O-, -S-, -CO-, -O-CO-, -CO-O-, -S-CO-, -CO-S-, -CH=CH-COO-, -CH=CH-, -CHhalogen and/or -CHCN-, or are a single bond, |
| X | is halogen, CN or $CH_3$, |

| | |
|---|---|
| $R^o$ | is H or an alkyl group having 1 to 10 C atoms which is different from X and $-Q^2-R^2-$, |
| $R^2$ | is an alkyl group having two to 10 C atoms, and |
| C* | is a carbon atom which is linked to four different substituents. |

**3.** Chiral derivatives of 1,2-difluorobenzene of the formula I according to Claim 1, characterised in that Q* is a radical of the formula

$Q^1-C^*R^oX-Q^2-R^2$

in which

| | |
|---|---|
| $Q^1$ and $Q^2$ | are each, independently of one another, alkylene having 1 to 2 C atoms, -O-, -O-CO-, -CO-O-, $-CH_2-O-$, $-O-CH_2-$ or a single bond, and |
| $R^o$, $R^2$, C* and X | have the meaning indicated in Claim 2. |

**4.** Compounds according to one of Claims 1 to 3, in which $-(A^1-Z^1)_m-PheF_2-(Z^2-A^2)_n-$ is a group of the formulae 1 to 15 (sic) below or a mirrow image thereof:

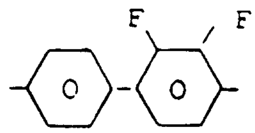

1

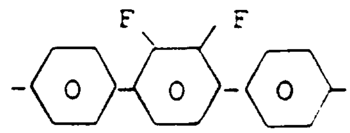

2

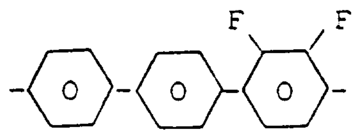

3

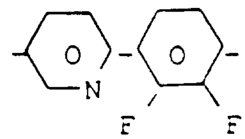

4

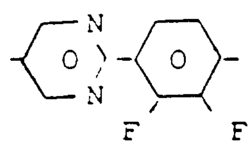

5

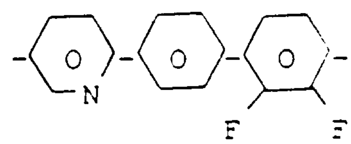

6

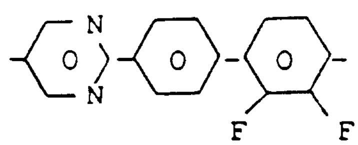

7

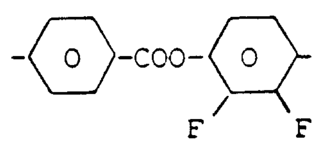

8

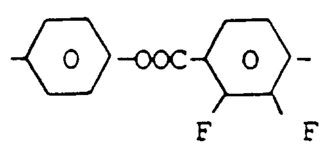

9

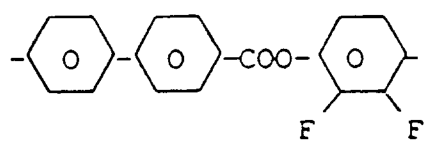

10

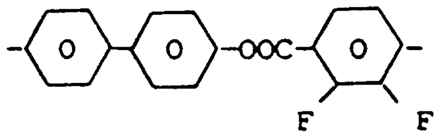

11

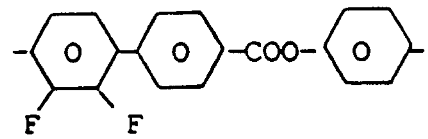

12

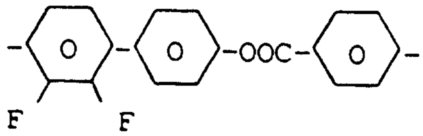

13

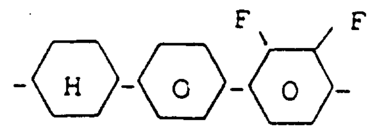

14

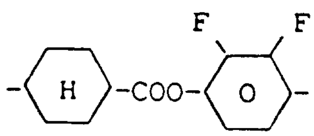

15

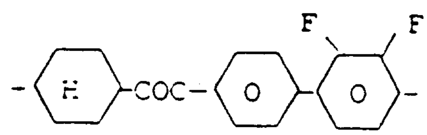

16

**5.** Compounds according to one of Claims 1 to 4, selected from the formulae A-M

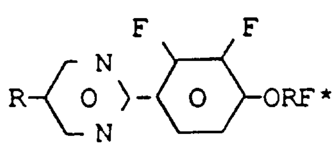

A

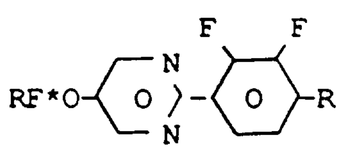

B

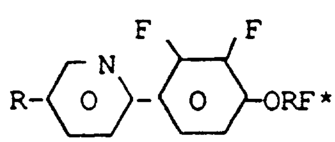

C

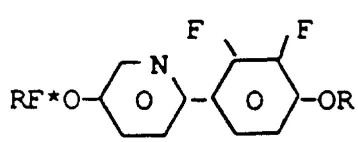

D

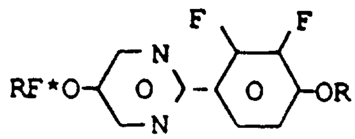

E

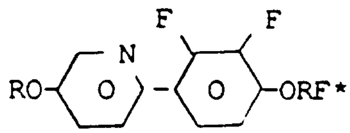

F

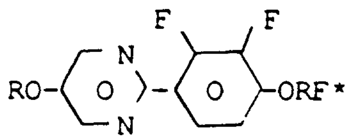

G

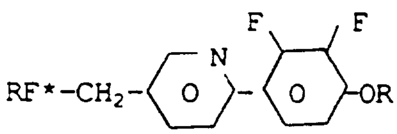

H

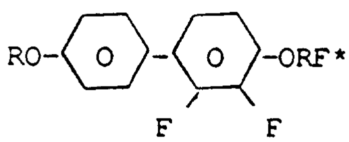

I

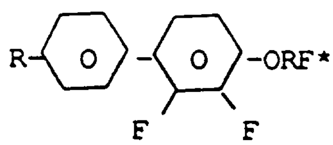

K

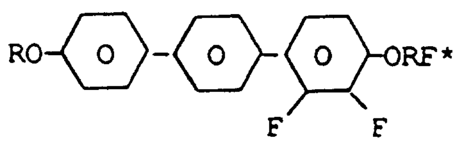

L

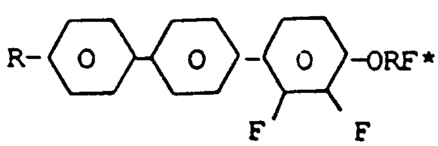

M

in which RF* is straight-chain or monobranched alkyl having 3 to 12 C atoms in which one $CH_2$ group has been replaced by -C*HF-, and C* is an asymmetric C atom, preferably

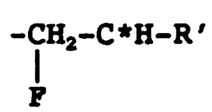

in which R' is straight-chain or monobranched alkyl, preferably having 2 to 10, in particular 3 to 10, C atoms, and R has one of the meanings of $R^1$.

**6.** Compounds according to one of Claims 1 to 5 of the formula A'

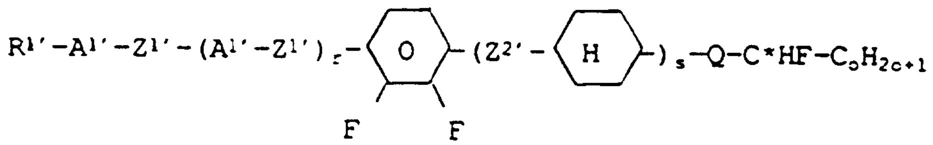 A'

in which

- $R^{1'}$ is an alkyl or alkenyl radical having up to 12 C atoms which is unsubstituted, monosubstituted by -CN or at least monosubstituted by fluorine or chlorine, it also being possible for one $CH_2$ group in these radicals to have been replaced by -O-, -CO- or -CO-O-,
- $A^{1'}$ in each case independently of one another, are a 1,4-phenylene radical which is unsubstituted or substituted by one or two fluorine atoms, or a pyridine-2,5-diyl radical, a pyrimidine-2,5-diyl radical, a pyrazine-2,5-diyl radical, a pyridazine-3,6-diyl radical, a 1,3,4-thiadiazole-2,5-diyl radical, a trans-1,4-cyclohexylene radical in which, in addition, one or two non-adjacent $CH_2$ groups may be replaced by -O- and/or -S-, a 1,4-bicyclo(2.2.2)octylene radical or a piperidine-1,4-diyl radical,
- $Z^{1'}$ and $Z^{2'}$ in each case independently of one another, are -CO-O-, -O-CO-, $-CH_2O-$, $-OCH_2-$, $-CH_2CH_2-$, -C = C-or a single bond,
- Q is $-OCH_2-$, $-COOCH_2-$ or $-CH_2OCH_2-$,
- o is 1 to 12,

and one of the two values

- r and s is 0 and the other is 0 or 1.

**7.** Chiral tilted smectic liquid-crystalline phase having at least two liquid-crystalline components, characterised in that it contains at least one compound of the formula I according to Claim 1.

**8.** Phase according to Claim 7, characterised in that it contains at least one compound of the sub-formulae IIa to IIg:

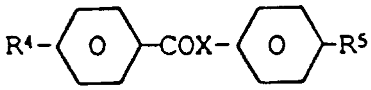

IIa

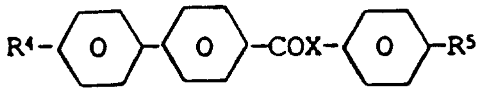

IIb

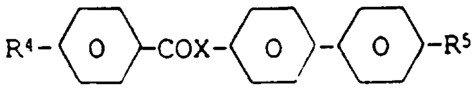

IIc

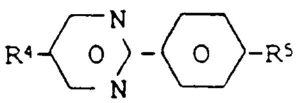

IId

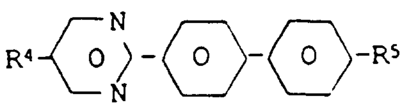

IIe

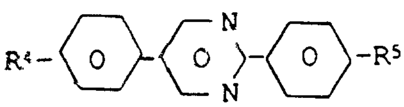

IIf

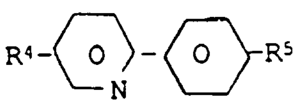

IIg

in which $R^4$ and $R^5$ are both alkyl, alkoxy, alkanoyloxy or alkoxycarbonyl, in each case having 3 to 12 carbon atoms, and X is 0 (sic), it also being possible for one 1,4-phenylene group to be laterally substituted by halogen, particularly preferably by fluorine.

**9.** Phase according to Claim 8, characterised in that it, besides components of the formulae IIa to IIg, also contains at least one compound of the formulae IIIa to IIIc:

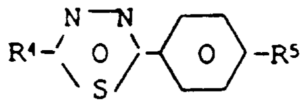

IIIa

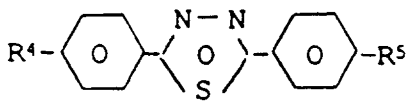

IIIb

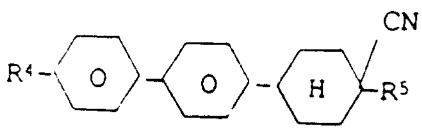

IIIc

in which $R^4$ and $R^5$ have the meaning given in the case of the formulae IIa to IIg.

**10.** Use of the compounds of the formula I according to Claim 1 as components of liquid-crystalline phases.

**11.** Ferroelectric electrooptical display element, characterised in that it contains, as dielectric, a phase according to Claim 7.

**Revendications**

**1.** Dérivés chiraux du 1,2-difluorobenzène de formule I,

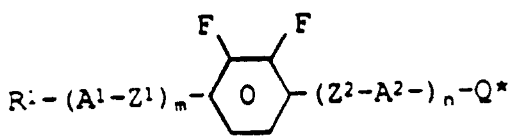

I

dans laquelle

$R^1$ représente H, F, Cl, Br, CN, un groupe alcoyle ou perfluoroalcoyle avec à chaque fois de 1 à 12 atomes de carbone, où également 1 ou 2 groupes $CH_2$ ou $CF_2$ non-voisins peuvent être représentés par des atomes de O et/ou des groupes -CO- et/ou des groupes -CO-O- et/ou des groupes -CH=CH- et/ou des groupes - CHHalogènes, et/ou des groupes -CO-O-CHCN-, ou un radical optiquement actif de formule

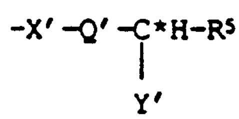

dans laquelle

X' représente -CO-O-, -O-CO-, -C-CO-O, -CO-, -O--S-, -CH=CH-, -CH=CH-COO- ou une liaison simple,

Q' représente un alcoylène en $C_{1-5}$ où également un groupe $CH_2$ non-relié par X' peut être remplacé par -O-, -CO-, -O-CO-, ou -CH=CH-, ou une liaison simple,

Y' représente CN, un halogène ou un méthyle, et

$R^5$ représente un groupe alcoyle différent de Y', ayant de 1 à 15 atomes de carbone, où également un ou plusieurs groupes $CH_2$ non voisins peuvent être remplacés par -O-, -CO-, -O-CO-, -CO-O-, et/ou -CH = CH-,

$A^1$ et $A^2$ représentent chacun indépendamment l'un de l'autre un 1,4-phénylène non-substitué ou substitué par un ou deux atomes de F et/ou Cl et/ou groupes $CH_3$ et/ou groupes CN, où également un ou deux groupes CH peuvent être remplacés par N, un 1,4-cyclohexylène où également un ou deux groupes $CH_2$ non voisins peuvent être remplacés par des atomes de O et/ou des atomes de S, un pipéridine-1,4-diyle, 1,4-bicyclo(2,2,2)octylène, 1,3,4-thiadiazol-2,5-diyle, naphtalène-2,6-diyle, décahydronaphtalène-2,6-diyle, ou 1,2,3,4-tétrahydronaphtalène-2,6-diyle,

$Z^1$ et $Z^2$ représentent chacun -CO-O-, -O-CO-, $-CH_2CH_2-$, $-OCH_2-$, $-CH_2O-$, -C≡C- ou une liaison simple,

m vaut 0, 1 ou 2,

n vaut 0, 1 ou 2,

(m + n) vaut 1 ou 2, et

Q* représente un radical organique inducteur de chiralité ayant un atome de carbone asymétrique.

**2.** Dérivés chiraux du 1,2-difluorobenzène de formule I selon la revendication 1, caractérisés en ce que Q* représente un radical de formule

$Q^1$-C*$R^o$X-$Q^2$-$R^2$

dans laquelle

$Q^1$ et $Q^2$ représentent à chaque fois indépendamment l'un de l'autre un alcoylène en $C_{2-4}$, où également un groupe $CH_2$ peut être remplacé par -O-, -S-, -CO-, -O-CO-, -CO-O-, -S-CO-, -CO-S-, -CH = CH-COO-, -CH = CH-, -CHHalogène, et/ou -CHCN-ou une liaison simple,

X représente un halogène, CH ou $CH_3$,

$R^o$ représente H ou un groupe alcoyle différent de X et de -$Q^2$-$H^2$, en $C_{1-10}$,

$R^2$ représente un groupe alcoyle en $C_{2-10}$ et

C* représente un atome de carbone relié à quatre substituants différents.

**3.** Dérivés chiraux du 1,2-difluorobenzène de formule I selon la revendication 1, caractérisés en ce que Q* représente un radical de formule

$Q^1$-C*$R^o$X-$Q^2$-$R^2$

dans laquelle

$Q^1$ et $Q^2$ représentent chacun indépendamment l'un de l'autre un alcoylène en $C_{1-2}$, -O-, -O-CO-, -CO-O-, $-CH_2-O-$, $-O-CH_2-$ ou une liaison simple, et

$R^o$, $R^2$, C* et X ont la signification donnée dans la revendication 2.

**4.** Composés selon l'une des revendications 1 à 3, où $(A^1-Z^1)_m-PheF_2-(Z^2-A^2)_n-$ représente un groupe de formules 1 à 15 ci-dessous, ou son image spéculaire :

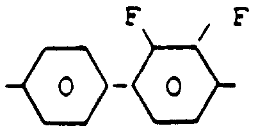

1

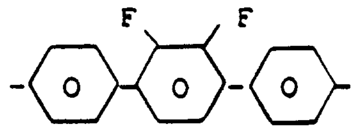

2

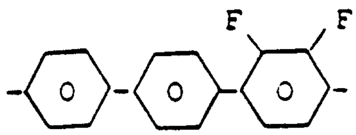

3

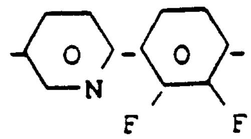

4

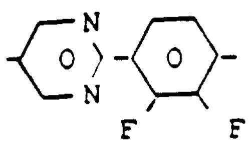

5

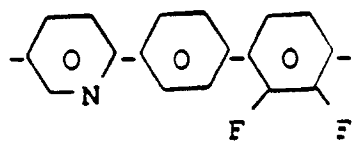

6

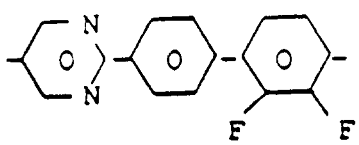

7

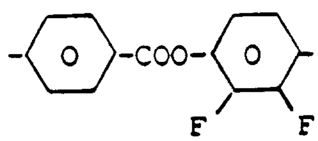

8

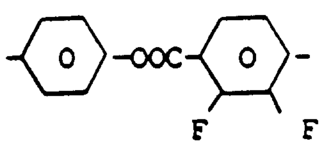

9

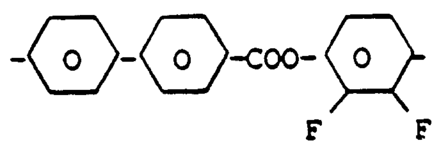

10

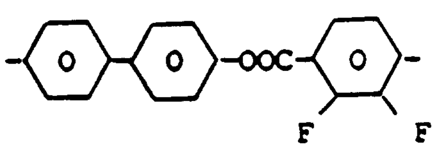

11

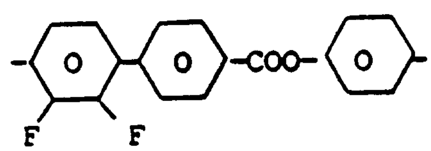

12

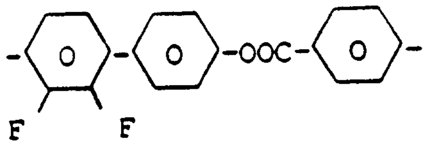

13

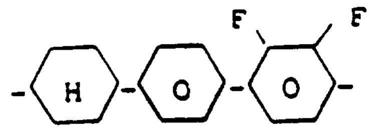

14

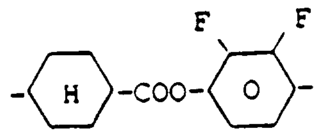

15

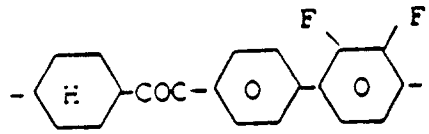

16

**5.** Composés selon l'une des revendications 1 à 4, choisis parmi les formules A-M :

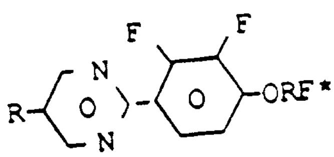

A

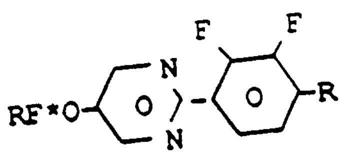

B

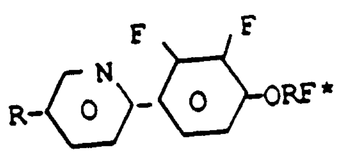

C

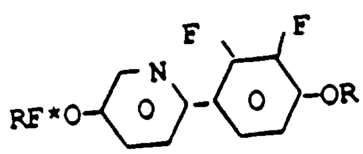

D

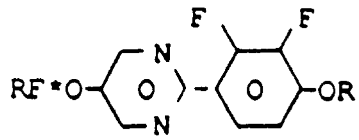 E

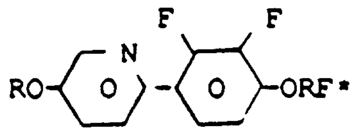 F

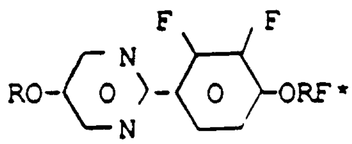 G

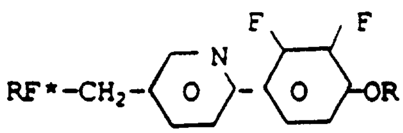 H

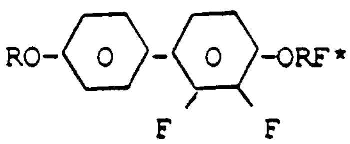 I

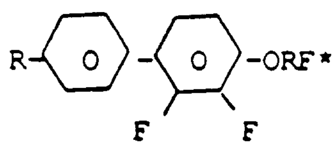 K

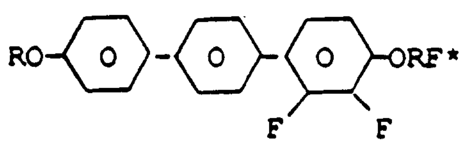 L

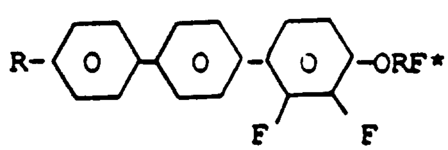 M

où RF* représente un alcoyle en $C_{3-12}$ à chaîne droite ou monoramifiée où un groupe $CH_2$ est remplacé par -C*HF- et C* est un atome de carbone asymétrique, de préférence

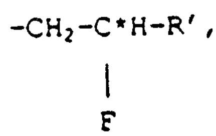

où

R' représente un alcoyle à chaîne droite ou monoramifiée ayant de préférence de 2 à 10, en

particulier de 3 à 10, atomes de carbone, et R présente l'une des significations de $R^1$.

6. Composés selon l'une des revendications 1 à 5 de formule A'

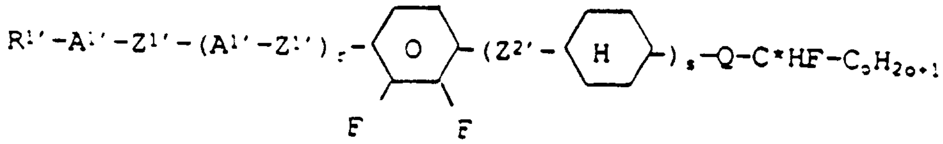

A'

dans laquelle

$R^{1'}$ représente un radical alcoyle ou alcényle non-substitué, monosubstitué par -CN ou au moins monosubstitué par un fluor ou un chlore et ayant jusqu'à 12 atomes de carbone où également dans ces radicaux un groupe $CH_2$ peut être remplacé par -O-, -CO- ou -CO-O-,

$A^{1'}$ représente à chaque fois indépendamment un radical 1,4-phénylène, un radical pyridine-2,5-diyle, un radical pyrimidine-2,5-diyle, un radical pyrazine-2,5-diyle, un radical pyridazine-3,6-diyle, un radical 1,3,4-thiadiazol-2,5-diyle, un radical trans-1,4-cyclohexylène, non-substitué ou substitué par 1 ou 2 atomes de fluor, où également un ou deux groupes $CH_2$ non-voisins peuvent être remplacés par -O- et/ou -S-, un radical 1,4-bicyclo(2.2.2)octylène ou un radical pipéridin-1,4-diyle,

$Z^{1'}$ et $Z^{2'}$ représentent chacun indépendamment l'un de l'autre -CO-O-, -O-CO-, $-CH_2O-$, $-OCH_2-$, $-CH_2CH_2-$, -C≡C- ou une liaison simple,

Q représente $-OCH_2-$, $-COOCH_2-$, ou $-CH_2OCH_2-$,

o vaut de 1 à 12,

et l'une des deux valeurs

r et s vaut 0 et l'autre vaut 0 ou 1.

7. Phase cristalline liquide smectique à inclinaison chirale avec au moins deux composants à cristaux liquides, caractérisée en ce qu'elle contient au moins un composé de formule I selon la revendication 1.

8. Phase selon la revendication 7, caractérisée en ce qu'elle contient au moins un composé de formules partielles IIa à IIg :

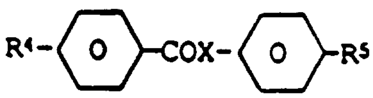

IIa

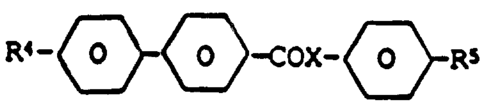

IIb

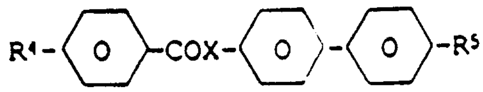

IIc

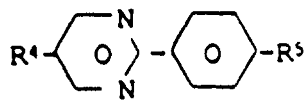

IId

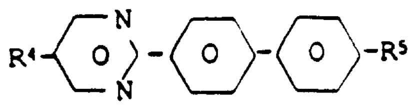

IIe

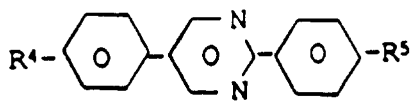

IIf

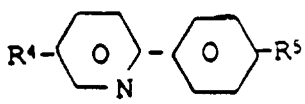

IIg

où $R^4$ et $R^5$ représentent chacun un alcoyle, alcoxy, alcanoyloxy ou alcoxycarbonyle avec à chaque fois de 3 à 12 atomes de carbone, et X vaut 0, où également un groupe 1,4-phénylène peut être substitué latéralement par un halogène, en particulier de préférence par un fluor.

**9.** Phase selon la revendication 8, caractérisée en ce qu'elle contient encore, outre les composants de formules IIa à IIg, au moins un composé de formules IIa à IIIc :

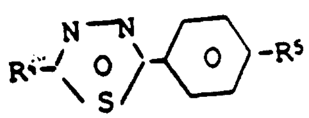

IIIa

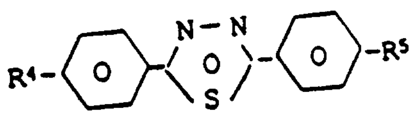

IIIb

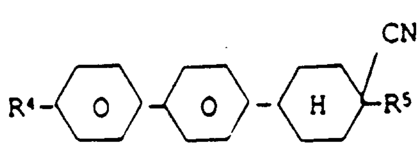

IIIc

où $R^4$ et $R^5$ ont les significations indiquées pour les formules IIa à IIg.

**10.** Application des composés de formule I selon la revendication 1 comme composants de phases à cristaux liquides.

11. Elément d'affichage électrooptique ferroélectrique, caractérisé en ce qu'il contient comme diélectrique une phase selon la revendication 7.